(19) Europäisches Patentamt
European Patent Office
Office européen des brevets

(11) Veröffentlichungsnummer: **0 283 857 B1**

(12) # EUROPÄISCHE PATENTSCHRIFT

(45) Veröffentlichungstag der Patentschrift: **18.03.92**

(51) Int. Cl.[5]: **C07D 233/61**, C07D 233/88, C07D 233/64, A61K 31/415

(21) Anmeldenummer: **88103746.9**

(22) Anmeldetag: **09.03.88**

(54) **Benzylidenamino-, benzylamino- und benzoylamino-imidazolderivate.**

(30) Priorität: **20.03.87 CH 1073/87**

(43) Veröffentlichungstag der Anmeldung:
**28.09.88 Patentblatt 88/39**

(45) Bekanntmachung des Hinweises auf die Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 0 200 947**
**FR-A- 2 487 830**

**CHEMICAL ABSTRACTS, Band 107, Nr. 7, 17. August 1987, Columbus, Ohio, USA; COCCO, M.T.; CONGIU, C.; PLUMITALLO, A.; SCHIVO, M.L.; PALMIERI, G.: "Synthesis and antifungal activities of N-arylidene-aminoimidazole derivatives." Seite 426, Spalte 2, Zusammenfassung-Nr. 55 624q**

(73) Patentinhaber: **F. HOFFMANN-LA ROCHE AG**
**Postfach 3255**
**CH-4002 Basel(CH)**

(72) Erfinder: **Klötzer, Wilhelm, Prof. Dr.**
**Löfflerweg 5**
**A-6020 Innsbruck(AT)**
Erfinder: **Montavon, Marc, Dr.**
**Realpstrasse 72**
**CH-4054 Basel(CH)**
Erfinder: **Müssner, Renate, Dr.**
**Bachweg 11**
**FL-9485 Nendeln(LI)**
Erfinder: **Singewald, Nicolas**
**Unterbergerstrasse 14**
**A-6020 Innsbruck(AT)**

(74) Vertreter: **Lederer, Franz, Dr. et al**
**Patentanwalt Dr. Franz Lederer Lucile-Grahn-Strasse 22**
**W-8000 München 80(DE)**

**EP 0 283 857 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Imidazolderivate. Im speziellen betrifft sie Imidazolderivate der allgemeinen Formel

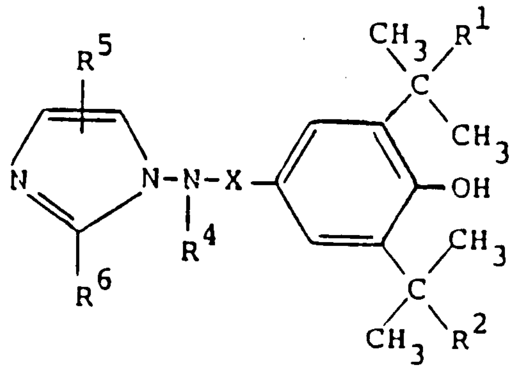

I

worin $R^1$ und $R^2$ je ($C_1$-$C_7$)-Alkyl, X einen Rest der Formel

$-CH(R^3)-$ oder $-CO-$ ;

(a) (b)

$R^3$ Wasserstoff und $R^4$ Wasserstoff oder ($C_1$-$C_7$)-Alkyl oder $R^3$ und $R^4$ zusammen eine zusätzliche Kohlenstoff-Stickstoff-Bindung; $R^5$ Wasserstoff oder ($C_1$-$C_7$)-Alkyl; $R^6$ Wasserstoff, ($C_1$-$C_7$)-Alkyl, ($C_1$-$C_7$)-Alkylthio oder einen Rest der Formel $-NR^7R^8$; und $R^7$ und $R^8$ je ($C_1$-$C_7$)-Alkyl oder zusammen mit dem Stickstoffatom einen 5- oder 6-gliedrigen gesättigten Heterocyclus bedeuten;
und pharmazeutisch annehmbare Säureadditionssalze davon.

Diese Verbindungen sind neu, und es hat sich gezeigt, dass sie wertvolle pharmakodynamische Eigenschaften besitzen, insbesondere entzündungshemmende und oedemhemmende Eigenschaften, sodass sie zur Bekämpfung oder Verhütung von Krankheiten verwendet werden können, insbesondere zur Bekämpfung oder Verhütung von Entzündungen und Oedemen.

Gegenstand der vorliegenden Erfindung sind die eingangs definierten Verbindungen und Salze als solche und als therapeutische Wirkstoffe, die Herstellung dieser Verbindungen und Salze, ferner Arzneimittel, enthaltend eine solche Verbindung oder ein Salz davon, die Herstellung solcher Arzneimittel, sowie die Verwendung der eingangs definierten Verbindungen und Salze zur Herstellung von Arzneimitteln gegen Entzündungen und Oedeme.

Der Ausdruck "Alkyl" bezeichnet geradkettige oder verzweigte gesättigte Kohlenwasserstoffreste mit vorzugsweise 1-4 Kohlenstoffatomen, wie Methyl, Aethyl, n-Propyl, Isopropyl, n-Butyl, Isobutyl, sek.-Butyl, tert.-Butyl und dergleichen. Der Ausdruck "Alkylthio" bezeichnet einen über ein Schwefelatom gebundenen Alkylrest im Sinne der vorstehenden Definition des Ausdrucks "Alkyl". Der Ausdruck "5- oder 6-gliedriger gesättigter Heterocyclus" umfasst Reste wie 1-Pyrrolidinyl, Piperidino, Morpholino und dergleichen.

In Formel I können zweckmässigerweise $R^1$ und $R^2$ je Methyl, $R^5$ Wasserstoff oder Methyl in 4-Stellung, $R^6$ Wasserstoff, Methyl, n-Propyl, Isopropyl, Methylmercapto oder Morpholino und $R^3$ Wasserstoff und $R^4$ Wasserstoff oder Methyl oder $R^3$ und $R^4$ zusammen eine zusätzliche Kohlenstoff-Stickstoff-Bindung bedeuten.

Besonders bevorzugte Verbindungen der Formel I sind:
1-(4-Hydroxy-3,5-di-tert.-butylbenzylamino)imidazol;
1-(4-Hydroxy-3,5-di-tert.-butylbenzylidenamino)-2-methylimidazol und
1-(4-Hydroxy-3,5-di-tert.-butylbenzylidenamino)imidazol.

Ebenfalls bevorzugte Verbindungen der Formel I sind:
1-(4-Hydroxy-3,5-di-tert.-butylbenzylamino)-2-methylimidazol;
1-(4-Hydroxy-3,5-di-tert.-butylbenzylamino)-2-propylimidazol und
1-(4-Hydroxy-3,5-di-tert.-butylbenzylamino)-2-methylmercaptoimidazol.

Weitere repräsentative Verbindungen der Formel I sind:
1-(4-Hydroxy-3,5-di-tert.-butylbenzoylamino)-2-methylimidazol;
1-(4-Hydroxy-3,5-di-tert.-butylbenzoylamino)imidazol;
1-(4-Hydroxy-3,5-di-tert.-butylbenzylidenamino)-2-morpholinoimidazol;
1-(4-Hydroxy-3,5-di-tert.-butylbenzylamino)-2-morpholinoimidazol;
1-(4-Hydroxy-3,5-di-tert.-butylbenzylidenamino)-2-isopropylimidazol;
1-(4-Hydroxy-3,5-di-tert.-butylbenzylidenamino)-4-methylimidazol;
1-(4-Hydroxy-3,5-di-tert.-butylbenzylamino)-4-methylimidazol;
1-(4-Hydroxy-3,5-di-tert.-butylbenzylidenamino)-2-propylimidazol; und
1-[N-(4-Hydroxy-3,5-di-tert.-butylbenzyl)-N-methylamino]-imidazol.

Die Imidazolderivate der eingangs definierten Formel I und deren pharmazeutisch annehmbare Säureadditionssalze können erfindungsgemäss dadurch hergestellt werden, dass man

a) zur Herstellung einer Verbindung der Formel I, Worin X -CH($R^3$)- und $R^3$ und $R^4$ zusammen eine zusätzliche Kohlenstoff-Stickstoff-Bindung bedeuten,
eine Verbindung der allgemeinen Formel

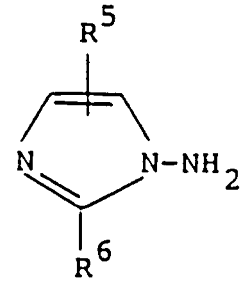

II

worin $R^5$ und $R^6$ obige Bedeutung besitzen,
mit einem Aldehyd der allgemeinen Formel

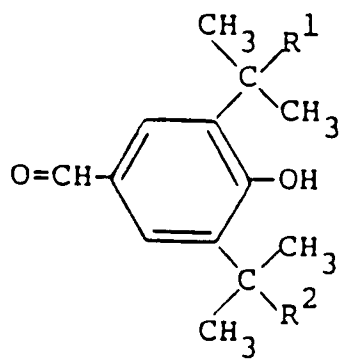

III

worin $R^1$ und $R^2$ obige Bedeutung besitzen,
kondensiert; oder

b) zur Herstellung einer Verbindung der Formel I, worin X -CO- und $R^4$ Wasserstoff oder ($C_1$-$C_7$)-Alkyl bedeuten,
eine Verbindung der allgemeinen Formel

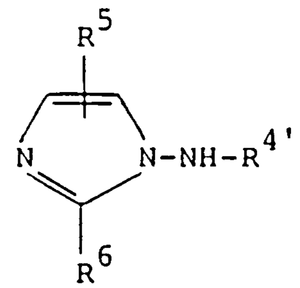

IV

worin $R^5$ und $R^6$ obige Bedeutung besitzen und $R^{4'}$ Wasserstoff oder $(C_1-C_7)$-Alkyl bedeutet, mit einem reaktionsfähigen Derivat einer Carbonsäure der allgemeinen Formel

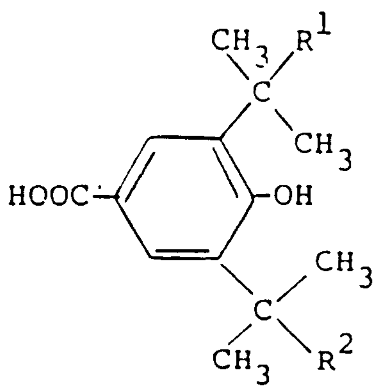

V

worin $R^1$ und $R^2$ obige Bedeutung besitzen, acyliert; oder

c) zur Herstellung einer Verbindung der Formel I, worin X $-CH_2-$ und $R^4$ Wasserstoff bedeuten, eine Verbindung der allgemeinen Formel

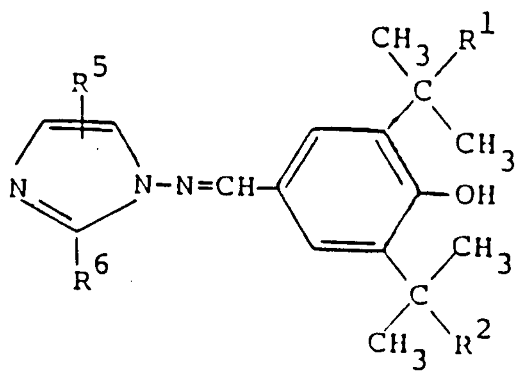

Ia

worin $R^1$, $R^2$, $R^5$ und $R^6$ obige Bedeutung besitzen, reduziert; oder

d) zur Herstellung einer Verbindung der Formel I, worin X $-CH_2-$ und $R^4$ Wasserstoff oder $(C_1-C_7)$-Alkyl bedeuten,

eine Verbindung der allgemeinen Formel

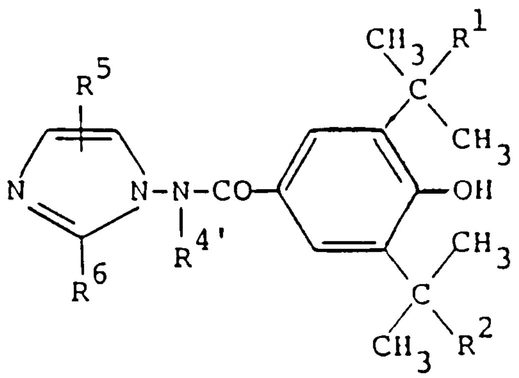

Ib

worin $R^1$, $R^2$, $R^{4'}$, $R^5$ und $R^6$ obige Bedeutung besitzen,
reduziert; oder
e) zur Herstellung einer Verbindung der Formel I, worin X $-CH_2-$ und $R^4$ $(C_1-C_7)$-Alkyl bedeuten,
eine Verbindung der allgemeinen Formel

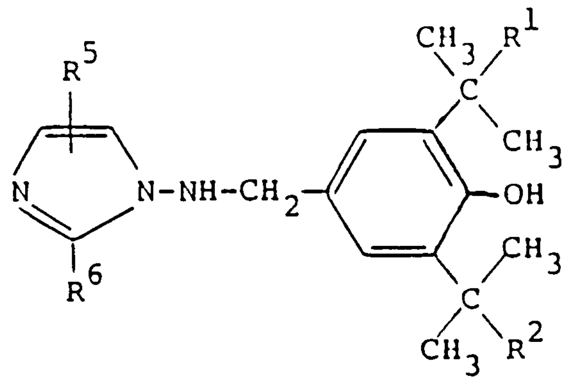

Ic

worin $R^1$, $R^2$, $R^5$ und $R^6$ obige Bedeutung besitzen,
entsprechend N-alkyliert; oder
f) zur Herstellung einer Verbindung der Formel I, worin X $-CH(R^3)-$ und $R^3$ und $R^4$ zusammen eine zusätzliche Kohlenstoff-Stickstoff-Bindung bedeuten,
eine Verbindung der obigen Formel Ic dehydriert; oder
g) zur Herstellung einer Verbindung der Formel I, worin X -CO- oder $-CH_2-$, $R^4$ Wasserstoff oder $(C_1-C_7)$-Alkyl und $R^6$ Wasserstoff bedeuten,
von einer Verbindung der allgemeinen Formel

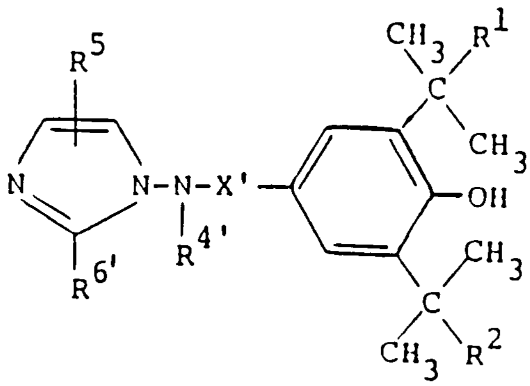

Id

worin $R^1$, $R^2$, $R^{4'}$ und $R^5$ obige Bedeutung
besitzen und X′ eine Methylen- oder Carbonylgruppe und $R^{6'}$ $(C_1-C_7)$-Alkylthio bedeuten,

die Alkylthiogruppe entfernt; oder

h) eine Verbindung der Formel I in ein pharmazeutisch annehmbares Säureadditionssalz überführt.

Für Aspekt a) des erfindungsgemässen Verfahrens verwendet man als Ausgangsprodukte der Formel II beispielsweise 1-Aminoimidazol, 1-Amino-2-methylimidazol und dergleichen, und als Ausgangsprodukt der Formel III beispielsweise 4-Hydroxy-3,5-di-tert.-butylbenzaldehyd. Die Umsetzung erfolgt in einem unter den Reaktionsbedingungen inerten organischen Lösungsmittel, zweckmässigerweise in einem niederen Alkanol, wie Methanol, Aethanol oder dergleichen. Weiterhin ist es zweckmässig, die Kondensation der Verbindungen der Formeln II und III in Gegenwart einer Säure durchzuführen; dies kann dadurch bewerkstelligt werden, dass man dem Reaktionsgemisch eine entsprechende Menge einer geeigneten Säure zufügt, beispielsweise Salzsäure, oder dass man das Ausgangsprodukt der Formel II in Form eines Säureadditionssalzes einsetzt, beispielsweise als Hydrochlorid. Die Kondensation erfolgt zweckmässigerweise bei Raumtemperatur und Normaldruck; die Reaktionszeit beträgt eine bis einige (maximal etwa 12) Stunden.

Als reaktionsfähige Derivate von Carbonsäuren der Formel V, welche in Aspekt b) des erfindungsgemässen Verfahrens als Ausgangsprodukte verwendet werden, eignen sich in erster Linie Säurehalogenide, insbesondere Säurechloride; es kommen aber auch andere reaktionsfähige Derivate in Betracht, beispielsweise gemischte Anhydride und dergleichen. Je nach der Natur des verwendeten reaktionsfähigen Derivates einer Carbonsäure der Formel V kann das Ausgangsprodukt der Formel IV als freie Base oder in Form eines Säureadditionssalzes eingesetzt werden, z.B. in Form des Hydrochlorids. Beispielsweise kann man so vorgehen, dass man 1-Aminoimidazol-hydrochlorid, 1-Amino-2-methylimidazol-hydrochlorid oder dergleichen ohne Zusatz eines Lösungsmittels mit 4-Hydroxy-3,5-di-tert.-butylbenzoylchlorid während etwa einer halben bis einer Stunde auf eine Temperatur von etwa 180-250 °C (zweckmässigerweise 200-210 °C) erhitzt, wobei das Gemisch unter Entwicklung von gasförmigem Chlorwasserstoff schmilzt.

Die Reduktion einer Verbindung der Formel Ia gemäss Aspekt c) des erfindungsgemässen Verfahrens kann beispielsweise durch katalytische Hydrierung erfolgen, wobei man zweckmässigerweise in Gegenwart eines Palladiumkatalysators oder dergleichen arbeitet. Die katalytische Hydrierung erfolgt zeckmässigerweise in einem unter den Reaktionsbedingungen inerten Lösungsmittel, z.B. in einem niederen Alkanol, wie Methanol oder dergleichen, oder in einem Gemisch eines mit Wasser mischbaren organischen Lösungsmittels und Wasser, z.B. in wässrigem Methanol oder dergleichen; der Zusatz einer Säure, wie Salzsäure, ist zweckmässig. Die katalytische Hydrierung wird zweckmässigerweise bei Raumtemperatur und Normaldruck durchgeführt; in der Regel ist die Hydrierung nach einer oder einigen Stunden beendet.

Die Reduktion der Verbindungen der Formel Ia kann aber auch mittels komplexer Hydride durchgeführt werden, beispielsweise mittels Natriumcyanoborhydrid oder dergleichen. Verwendet man als Reduktionsmittel Natriumcyanoborhydrid, dann kommt als Lösungsmittel in erster Linie Eisessig in Betracht. Bei dieser Ausführungsart der Reduktion wird zweckmässigerweise bei Raumtemperatur und Normaldruck gearbeitet. Die Reaktionszeit für eine Reduktion einer Verbindung der Formel Ia mittels eines komplexen Metallhydrids beträgt mehrere, zweckmässigerweise etwa 12 Stunden.

Die erfindungsgemässe Reduktion einer Verbindung der Formel Ia gemäss Aspekt c) des erfindungsgemässen Verfahrens liefert entsprechende Verbindungen der Formel Ic.

Die Reduktion einer Verbindung der Formel Ib gemäss Aspekt d) des erfindungsgemässen Verfahrens kann zweckmässigerweise mittels eines von Borwasserstoff abgeleiteten Reduktionsmittels erfolgen, beispielsweise mittels Boran-dimethylsulfidkomplex. Als Reaktionsmedium kommen wasserfreie, unter den Reaktionsbedingungen inerte organische Lösungsmittel oder Lösungsmittelgemische in Betracht, beispielsweise Gemische von Tetrahydrofuran und Toluol oder dergleichen. Die Reaktion erfolgt zweckmässigerweise bei erhöhter Temperatur, z.B. bei Rückflusstemperatur des Reaktionssystems, und die Reaktionsdauer beträgt einige (z.B. ca. 4) Stunden.

Die N-Alkylierung einer Verbindung der Formel Ic gemäss Aspekt e) des erfindungsgemässen Verfahrens wird zweckmässigerweise so durchgeführt, dass man das Ausgangsprodukt zunächst entsprechend N-acyliert und hierauf die so eingeführte Acylgruppe zur gewünschten Alkylgruppe reduziert. Will man also eine Verbindung der Formel Ic auf diese Weise N-methylieren, so führt man diese in einem ersten Schritt in die entsprechende N-Formylverbindung über, worauf man in einem zweiten Schritt die Formylgruppe zur Methylgruppe reduziert. Die N-Formylierung kann nach einer ersten Ausführungsart so durchgeführt werden, dass man Essigsäureanhydrid und Ameisensäure miteinander reagieren lässt, das gebildete gemischte Anhydrid mit einem geeigneten inerten organischen Lösungsmittel, wie Tetrahydrofuran oder dergleichen, aufnimmt, in die erhaltene Lösung eine Verbindung der Formel Ic einträgt und das Reaktionsgemisch eine bis einige Stunden bei Raumtemperatur stehen lässt. Nach einer anderen Ausführungsart kann man eine Verbindung der Formel Ic in Ameisensäure lösen, hierauf Essigsäureanhydrid zugeben und das Gemisch über Nacht stehen lassen.

Die Reduktion der so erhaltenen N-Formylverbindung erfolgt zweckmässigerweise mittels eines von

Borwasserstoff abgeleiteten Reduktionsmittels, beispielsweise mittels Boran-dimethylsulfidkomplex. Diese Reduktion erfolgt zweckmässigerweise in einem wasserfreien, unter den Reaktionsbedingungen inerten organischen Lösungsmittel oder Lösungsmittelgemisch, z.B. in einem Gemisch von Tetrahydrofuran und Toluol oder dergleichen. Die Reduktion dauert einige (ca. 4) Stunden und erfolgt zweckmässigerweise bei erhöhter Temperatur, z.B. bei Rückflusstemperatur des Reaktionssystems.

Die Dehydrierung einer Verbindung der Formel Ic gemäss Aspekt f) des erfindungsgemässen Verfahrens liefert eine entsprechende Verbindung der Formel Ia und erfolgt unter Verwendung von Mitteln, welche für derartige Dehydrierungen gebräuchlich sind, wie Dicyanodichlorbenzochinon, Chloranil, Azodicarbonsäurediäthylester und dergleichen. Die Reaktionsbedingungen können je nach eingesetztem Dehydrierungsmittel variieren, sind jedoch im Prinzip jedem Fachmann geläufig. Verwendet man beispielsweise als Dehydrierungsmittel Dicyanodichlorbenzochinon so arbeitet man zweckmässigerweise in einem unter den Reaktionsbedingungen inerten organischen Lösungsmittel, beispielsweise in einem aromatischen Kohlenwasserstoff, wie Benzol, Toluol, Xylol oder dergleichen, vorteilhafterweise in Gegenwart geringer Mengen einer wasserfreien Säure, wie Essigsäure oder dergleichen. Die Dehydrierung dauert etwa 10 Minuten bis 1 Stunde und erfolgt bei erhöhter Temperatur, zweckmässigerweise bei oder etwas unterhalb der Rückflusstemperatur des Reaktionssystems.

Die Abspaltung einer niederen Alkylthiogruppe aus einer Verbindung der Formel Id gemäss Aspekt g) des erfindungsgemässen Verfahrens wird unter Verwendung allgemein üblicher und dem Fachmann geläufiger Methoden durchgeführt. Beispielsweise löst man eine Verbindung der Formel Id in einem unter den Reaktionsbedingungen inerten organischen Lösungsmittel, beispielsweise in einem niederen Alkanol, wie Methanol, gibt dann ein Nickel(II)salz, wie Nickel(II)chlorid$\cdot 6H_2O$, und anschliessend eine komplexe Bor-Wasserstoff-Verbindung wie Natriumborhydrid, zu und lässt dann das Reaktionsgemisch bei Raumtemperatur stehen, bis die Reduktion beendet ist, was einige (ca. 2-5) Stunden dauert. Die Abspaltung einer niederen Alkylthiogruppe aus einer Verbindung der Formel Id kann aber z.B. auch mittels Raney-Nickel erfolgen, zweckmässigerweise in Aethanol o.dgl. bei einer Temperatur von etwa 50 bis 75 °C, was ebenfalls einige (z.B. 2) Stunden dauert.

Gemäss Aspekt h) des erfindungsgemässen Verfahrens können Verbindungen der Formel I durch Umsetzung mit organischen oder anorganischen Säuren in entsprechende Säureadditionssalz übergeführt werden, wobei im Rahmen der vorliegenden Erfindung die Verwendung solcher Säuren in Betracht zu ziehen ist, welche pharmazeutisch annehmbare Salze bilden, beispielsweise Chlorwasserstoff, Bromwasserstoff, Phosphorsäure, Schwefelsäure, Zitronensäure, p-Toluolsulfonsäure und dergleichen.

Die Ausgangsprodukte der Formel II sind bekannt oder nach an sich bekannten und jedem Fachmann geläufigen Methoden leicht herstellbar; zudem enthalten manche der nachfolgenden Beispiele detaillierte Angaben betreffend die Herstellung bestimmter Verbindungen der Formel II. Verbindungen der Formel IV, worin $R^{4'}$ niederes Alkyl bedeutet, können durch N-Alkylierung entsprechender Verbindungen der Formel II hergestellt werden, und zwar in Analogie zur N-Alkylierung von Verbindungen der Formel Ic gemäss Variante e) des erfindungsgemässen Verfahrens.

Wie eingangs erwähnt, besitzen die Imidazolderivate der allgemeinen Formel I und deren pharmazeutisch annehmbare Säureadditionssalze wertvolle pharmakodynamische Eigenschaften.

Repräsentative Verbindungen der Formel I wurden im nachfolgend beschriebenen Tierversuch auf ihre entzündungshemmenden Eigenschaften untersucht:

Männlichen Ratten (120-140 g) injiziert man in die Schwanzwurzel 0,1 ml einer 0,5%igen (Gewicht/Volumen) Suspension von durch Erhitzen abgetötetem und getrocknetem Mycobacterium butyricum im schwerem Mineralöl, enthaltend 0,2% Digitonin. Die Tiere werden einzeln gehalten und erhalten Futter und Wasser ad libitum. Man lässt die so erzeugte Arthritis sich ohne Behandlung während 21 Tagen entwickeln und ermittelt dann einerseits das Körpergewicht der Tiere und andererseits das Volumen ihrer beiden Hinterpfoten (und zwar durch Eintauchen der Pfoten in einen Quecksilberplethysmographen bis zur Höhe des lateralen Malleolus). Hierauf teilt man die Tiere in je sechs Tiere umfassende Gruppen von jeweils unfähr gleichen mittleren Volumina der Hinterpfoten und verabreicht ihnen dann die zu untersuchende Substanz über einen Zeitraum von 7 Tagen täglich durch Intubation. Am Ende der Behandlungsperiode werden Körpergewicht und Volumen der Hinterpfoten erneut ermittelt und deren Veränderung über den Behandlungszeitraum berechnet. Anschliessend tötet man die Tiere und entnimmt ihnen Plasmaproben zur Ermittlung des Fibrinogen gemäss Exner et al., 1979 (Amer. J. Clin. Path.) nach Ausfällung mit Ammoniumsulfat.

In der nachfolgenden Tabelle sind für drei repräsentative Verbindungen der eingangs definierten Formel I die in dem vorstehend beschriebenen Tierversuch ermittelten Resultate wiedergegeben.

| Verbindung | Dosis (µmol/kg) | Entzündungshemmende Wirkung | | Veränderung Körpergewicht (g) |
|---|---|---|---|---|
| | | Aenderung Pfotenvolumen (ml) | Plasma-Fibrinogen (mg/dl) | |
| Vehikel (Polysorbat 80) | - | 0,46 ± 0,08 | 1233 ± 56 | 13,2 ± 1,8 |
| A | 66 | -0,18 ± 0,08 * | 1040 ± 75 * | 30,5 ± 1,2 * |
| B | 66<br>22 | -0,84 ± 0,12 *<br>-0,77 ± 0,05 * | 75 ± 15*<br>405 ± 126 * | 35,3 ± 1,8 *<br>28,2 ± 1,4 * |
| C | 66 | -0,92 ± 0,08 * | 544 ± 97 * | 31,2 ± 2,0 * |

* Wert signifikant verschieden vom entsprechenden Wert für lediglich mit Vehikel behandelten Tieren ( $p<0,05$; Students t-Test).
A = 1-(4-Hydroxy-3,5-di-tert.-butylbenzylamino)imidazol.
B = 1-(4-Hydroxy-3,5-di-tert.-butylbenzylidenamino)-2-methylimidazol.
C = 1-(4-Hydroxy-3,5-di-tert.-butylbenzylidenamino)imidazol.

Die nachfolgende Tabelle enthält Angaben über die akute Toxizität der obigen drei Verbindungen ($DL_{50}$ bei einmaliger oraler Verabreichung an Mäuse).

| Verbindung | A | B | C |
|---|---|---|---|
| $DL_{50}$ (mg/kg p.o. | >1000 | >1000 | >1000 |

Weiterhin wurden die obige Verbindung A sowie die beiden folgenden, ebenfalls von Formel I umfassten Verbindungen

- D = 1-(4-Hydroxy-3,5-di-tert.-butylbenzylamino)-2-methylimidazol und
- E = 1-(4-Hydroxy-3,5-di-tert.-butylbenzylamino)-2-propylimidazol

im nachfolgend beschriebenen Tierversuch auf ihre oedemhemmenden Eigenschaften untersucht:

Bei männlichen Ratten (230-250 g), welche Futter und Wasser ad libitum erhalten, wird durch Injektion von 0,2 ml einer 1%igen Lösung von Carrageen in steriler, pyrogenfreier Kochsalzlösung in die rechte Pleurahöhle eine Pleuritis hervorgerufen. Die zu untersuchenden Substanzen, suspendiert in einem wässrigen Vehikel (enthaltend 0,5% Carboxymethylcellulose, 0,9% Kochsalz, 0,37% Polysorbat 80 und 0,86% Benzylalkohol), bzw. das Vehikel allein werden den Tieren 1 Stunde vor und 5 Stunden nach den Carrageen-Injektion durch Intubation verabreicht. 24 Stunden nach der Carrageen-Injektion tötet man die Tiere durch Enthauptung, lässt sie ausbluten und legt die Pleurahöhle frei, indem man die Rippen beiderseits des Brustbeins durchtrennt. Man entfernt das Exsudat mittels einer Pipette aus der Pleurahöhle und bestimmt dessen Volumen. Hierauf wäscht man die Pleurahöhle einmal mit phosphatgepufferter Kochsalzlösung, enthaltend foetales Rinderserum (1:1) und vereinigt die Waschlösung mit dem Exsudat. Unter Verwendung einer "Coulter Counter", der so eingestellt ist, dass rote Blutkörperchen nicht mitgezählt werden, wird die gesamte Anzahl von Zellen in der Pleurahöhle ermittelt. Zellabstriche auf Glasplättchen können direkt aus dem Exsudat gewonnen, in Methanol fixiert und zwecks Differentialzählung von polymorphonuclearen Leukozyten (PMN) und Makrophagen gefärbt werden; man kann insgesamt 200 PMN und Makrophagen auszählen und das Resultat als Prozentsatz jedes im Pleura-Exsudat vorkommenden Zellentyps ausdrücken.

Die Resultate des vorstehend beschriebenen Versuchs sind in der nachfolgenden Tabelle wiedergegeben:

| Verbindung | Dosis (mg/kg p.o.) | Veränderung in % im Vergleich zu Kontrollen | |
|---|---|---|---|
| | | Exsudatvolumen | Anzahl Zellen |
| A | 100<br>50<br>30 | -68*<br>-60*<br>-51* | -23*<br>-14*<br>-12 |
| D | 100<br>30 | -60*<br>-29* | -22*<br>- 6 |
| E | 100<br>30 | -73*<br>-39* | -21*<br>+ 4 |

* Wert signifikant verschieden vom entsprechenden Wert für lediglich mit Vehikel behandelten Tieren ($p<0,05$; Students t-Test)

Die Verbindungen der Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die Verbindungen der Formel I und ihre pharmazeutisch annehmbaren Säureadditionssalze mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I oder ein pharmazeutisch annehmbares Säureadditionssalz davon und ein therapeutisch inertes Excipiens, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe zusammen mit einem oder mehreren therapeutisch inerten Excipientien in eine galenische Darreichungsform bringt.

Wie eingangs erwähnt, können die Verbindungen der Formel I und ihre pharmazeutisch annehmbaren Säureadditionssalze erfindungsgemäss bei der Bekämpfung bzw. Verhütung von Krankheiten verwendet werden, und zwar insbesondere bei der Bekämpfung bzw. Verhütung von Entzündungen und Oedemen. Die Dosierung kann innerhalb weiter Grenzen variieren und ist natürlich in jedem einzelnen Fall den individuellen Gegebenheiten anzupassen. Im allgemeinen kommt bei oraler Verabreichung eine Tagesdosis von etwa 10 mg bis 2000 mg in Frage.

Ebenfalls Gegenstand der Erfindung ist, wie eingangs erwähnt, die Verwendung der Verbindungen der Formel I und ihrer pharmazeutisch annehmbaren Säureadditionssalze zur Herstellung von Arzneimitteln, insbesondere von Arzneimitteln gegen Entzündungen und Oedeme.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

a) 5 g Hydroxylamin-O-sulfonsäure werden in 30 ml Wasser gelöst und unter Eiskühlung mit 3,7 g Natriumbicarbonat neutralisiert. Diese Lösung wird zu einer Lösung von 3,63 g 2-Methylimidazol in 15 ml

Wasser zugetropft. Man rührt ca. 20 Stunden bei Raumtemperatur und säuert dann mit 13 ml 2N Salzsäure an, bis der pH der Lösung 1 beträgt. Man versetzt mit zwei Spatelspitzen Aktivkohle, rührt 15 Minuten, filtriert und versetzt das klare Filtrat mit 5 ml Benzaldehyd und 10 ml Aether. Hierauf rührt man 6 Stunden, kühlt dann 15 Minuten im Eisbad und filtriert den ausgefallenen Festkörper ab; das Filtrat wird wie weiter unten beschrieben behandelt.

Nach Umfällen des Festkörpers aus Methanol/Aether erhält man 1,3-Bis(benzylidenamino)-2-methylimidazoliumchlorid vom Smp. 244-249°.

Das obige Filtrat wird dreimal mit je 15 ml Aether ausgeschüttelt. Die wässrige Phase wird unter Kühlen mit 9 ml 4n Natronlauge neutralisiert (pH 7). Man filtriert den Niederschlag ab, fällt ihn aus Methanol/Aether um und erhält 1-Benzylidenamino-2-methylimidazol vom Smp. 122-124°.

b) 3,24 g 1,3-Bis(benzylidenamino)-2-methylimidazoliumchlorid werden in 50 ml Methanol gelöst. Die Lösung wird auf 0° (Innentemperatur) gekühlt und unter Rühren mit einer Lösung von 2,6 g Kaliumcyanid in 8 ml Wasser versetzt, wobei die Temperatur nicht wesentlich ansteigen darf. Nach 15 Minuten wird das Methanol bei möglichst tiefer Temperatur im Vakuum abgedampft; der Rückstand wird mit 30 ml Wasser verdünnt, worauf man dreimal mit Chloroform extrahiert. Der getrocknete Chloroformextrakt wird eingedampft, und man erhält 1-Benzylidenamino-2-methylimidazol vom Smp. 125°.

c) 7,4 g 1-Benzylidenamino-2-methylimidazol werden in 50 ml Wasser suspendiert. Die Suspension wird mit 25 ml 2N Salzsäure angesäuert (pH = 1-2) und dann solange einer Wasserdampfdestillation unterworfen, bis kein Benzaldehyd mehr übergeht. Die zurückbleibende klare Lösung wird zur Trockene eingedampft, und der ölige Eindampfrückstand wird mit Aether bei 0° zur Kristallisation gebracht. Die erhaltenen Kristalle werden aus Aethanol/Aether umgefällt, und man erhält 1-Amino-2-methylimidazol-hydrochlorid vom Smp. 139-141°.

d) 5,35 g 1-Amino-2-methylimidazol-hydrochlorid werden in 300 ml Aethanol gelöst, worauf man 9,3 g 4-Hydroxy-3,5-di-tert.-butylbenzaldehyd zugibt, 1 Stunde bei Raumtemperatur rührt und dann das Gemisch im Vakuum eindampft. Zum Rückstand gibt man 150 ml Eiswasser und 200 ml Methylenchlorid, rührt und neutralisiert (pH = 7) mit gesättigter Natriumbicarbonatlösung. Die Methylenchloridphase wird abgetrennt, und die wässerige Phase wird mit 100 ml Methylenchlorid extrahiert. Die vereinigten Methylenchloridphasen werden getrocknet und eingedampft. Man rührt den Rückstand mit Aether auf, filtriert, trocknet den Filterrückstand bei 80° im Vakuum und erhält 1-(4-Hydroxy-3,5-di-tert.-butylbenzylidenamino)-2-methylimidazol vom Smp. 206-207°.

## Beispiel 2

3,13 g 1-(4-Hydroxy-3,5-di-tert.-butylbenzylidenamino)-2-methylimidazol werden in 100 ml Methanol gelöst, worauf man 0,5 g Palladiumkatalysator (5% auf Kohle) und 10 ml 1N Salzsäure zugibt und dann bei Normaldruck und Raumtemperatur hydriert. Nachdem 240 ml Wasserstoff aufgenommen worden sind, filtriert man den Katalysator ab und dampft das Filtrat ein. Man nimmt den öligen Rückstand mit 50 ml Wasser auf und gibt bis zur neutralen Reaktion (pH = 7) gesättigte Natriumbicarbonatlösung zu. Der ausgefallene Rückstand wird abfiltriert und in 100 ml Methylenchlorid aufgenommen. Die Methylenchloridlösung wird mit 50 ml Wasser gewaschen, getrocknet und eingedampft. Man rührt den Rückstand mit Aether auf, filtriert und erhält rohes 1-(4-Hydroxy-3,5-di-tert.-butylbenzylidenamino)-2-methylimidazol vom Smp. 181-182°. Durch Umkristallisieren aus Acetonitril erhält man ein reines Produkt vom Smp. 182-183°.

## Beispiel 3

1,0 g 1-Amino-2-methylimidazol-hydrochlorid und 2,0 g 4-Hydroxy-3,5-di-tert.-butylbenzoylchlorid werden vermischt und auf dem Oelbad während 40 Minuten auf 200-210° (Badtemperatur) erhitzt, wobei der Kolbeninhalt unter HCl-Entwicklung schmilzt und dann wieder fest wird. Der abgekühlte Kolbeninhalt wird in 30 ml Wasser gelöst, worauf die Lösung filtriert und das Filtrat mit 10%iger Natriumcarbonatlösung auf pH 9 eingestellt wird. Der ausgefallene Niederschlag wird abfiltriert und in 25 ml heissem Aethanol gelöst, worauf man die noch heisse Lösung mit 10 ml Wasser versetzt. Nach Abkühlen wird der ausgefallene Festkörper abfiltriert und aus Essigester umrkristallisiert; man erhält 1-(4-Hydroxy-3,5-di-tert.-butylbenzoylamino)-2-methylimidazol vom Smp. 322-324° (Zers.).

## Beispiel 4

Eine Suspension von 200 mg 1-(4-Hydroxy-3,5-di-tert.-butylbenzoylamino) -2-methyl-1H-imidazol in 10 ml wasserfreiem Tetrahydrofuran wird unter Inertgas mit 1,2 ml einer 2N Boran-dimethylsulfidkomplexlösung in Toluol versetzt. Dann erhitzt man während 4 Stunden am Rückfluss, lässt auf 0° abkühlen, versetzt mit methanolischer Salzsäure (5 ml Methanol und 0,5 ml konz. HCl), erhitzt nochmals während 1 Stunde am Rückfluss und dampft im Vakuum ein. Man nimmt den Rückstand in 4 ml Wasser auf, gibt gesättigte Natriumbicarbonatlösung zu, bis der pH 8-9 beträgt, extrahiert dreimal mit je 5 ml Methylenchlorid und dampft die vereinigten Methylenchloridphasen nach Trocknen ein. Der Rückstand wird aus Cyclohexan umgelöst, und man erhält 1-(4-Hydroxy-3,5-di-tert.-butylbenzylamino)-2-methylimidazol vom Smp. 180°.

Beispiel 5

a) 22,6 g Hydroxylamin-O-sulfonsäure werden in 60 ml Eiswasser gelöst und mit ca. 18 g Natriumbicarbonat neutralisiert (pH = 6). Unter Rühren wird innerhalb von 45 Minuten eine Lösung von 27,2 g Imidazol in 60 ml Wasser zugetropft. Man rührt ca. 20 Stunden bei Raumtemperatur und säuert dann mit ca. 160 ml 2N Salzsäure unter Eiskühlung auf pH 1-2 an. Darauf rührt man die dunkle Lösung während 15 Minuten mit Aktivkohle, filtriert und versetzt das klare Filtrat mit 28 ml Benzaldehyd und 30 ml Aether. Hierauf rührt man 18 Stunden, kühlt dann die entstandene Suspension während 15 Minuten und filtriert das ausgefallene 1,3-Bis(benzylidenamino)imidazoliumchlorid ab.

Das Filtrat schüttelt man dreimal mit je 40 ml Aether aus. Dann neutralisiert man die wässrige, saure Phase unter Eiskühlung mit 40 ml 4N Natronlauge auf pH 7. Der ausgefallene Niederschlag wird einmal mit 100 ml Chloroform und noch zweimal mit je 20 ml Chloroform ausgeschüttelt. Die vereinigten Chloroformphasen werden über Natriumsulfat getrocknet und eingedampft. Der Eindampfrückstand wird unter Erwärmen in Aethanol gelöst, worauf man mit soviel heissem Wasser versetzt, dass keine Trübung eintritt. Anschliessend filtriert man rasch und lässt abkühlen. Nach 1-stündiger Eiskühlung filtriert man das ausgefallene 1-Benzylidenaminoimidazol ab.

b) In analoger Weise wie in Beispiel 1b) beschrieben erhält man aus 1,3-Bis(benzylidenamino)-imidazoliumchlorid 1-Benzylidenaminoimidazol vom Smp. 115°.

c) 8,68 g 1-Benzylidenaminoimidazol werden in 50 ml Wasser suspendiert, worauf man die Suspension mit 28 ml 2N Salzsäure ansäuert (pH = 1-2) und dann solange einer Wasserdampfdestillation unterwirft, bis kein Benzaldehyd mehr übergeht. Danach dampft man die zurückbleibende klare Lösung im Vakuum bei 50-60° zur Trockene ein und bringt den Eindampfrückstand bei 0° mit Aether zur Kristallisation. Nach Umfällen des Kristallisates aus Aethanol/Aether erhält man 1-Aminoimidazol-hydrochlorid vom Smp. 102°.

d) 0,48 g 1-Aminoimidazol-hydrochlorid werden in 30 ml Aethanol suspendiert, worauf man 0,93 g 4-Hydroxy-3,5-di-tert.-butylbenzaldehyd zugibt. Man rührt die Suspension 3 Stunden bei Raumtemperatur, wobei eine klare Lösung entsteht, welche im Vakuum eingedampft wird. Man nimmt den Rückstand in 15 ml Wasser auf, neutralisiert mit eiskalter gesättigter Natriumbicarbonatlösung (pH = 7) und schüttelt rasch dreimal mit je 30 ml Methylenchlorid aus. Die vereinigten organischen Phasen werden über Natriumsulfat getrocknet, filtriert und im Vakuum eingedampft. Durch Sublimation des Rückstands (160°/12 Torr) erhält man 1-(4-Hydroxy-3,5-di-tert.-butylbenzylidenamino)imidazol vom Smp. 171-174°.

Beispiel 6

0,9 g 1-(4-Hydroxy-3,5-di-tert.-butylbenzylidenamino)imidazol werden in 10 ml Eisessig suspendiert und mit 1,61 g Natriumcyanoborhydrid versetzt, wobei eine Lösung entsteht. Man rührt bei Raumtemperatur über Nacht und dampft dann im Vakuum ein. Man versetzt den Rückstand mit 10 ml Wasser, neutralisiert mit eiskalter gesättigter Natriumbicarbonatlösung (pH = 7) und schüttelt dreimal mit je 30 ml Methylenchlorid aus. Die vereinigten organischen Phasen werden über Natriumsulfat getrocknet, filtriert und im Vakuum eingedampft. Der Rückstand wird mit Aether zur Kristallisation gebracht. Das Produkt wird abfiltriert und aus Aethanol/Wasser umgefällt; man erhält 1-(4-Hydroxy-3,5-di-tert.-butylbenzylamino)imidazol vom Smp. 171-174°.

Beispiel 7

28,7 g 1-(4-Hydroxy-3,5-di-tert.-butylbenzylidenamino)imidazol werden in 500 ml Methanol gelöst, worauf man 96 ml 1N Salzsäure und 3 g Palladiumkohle zugibt und bei Normaldruck und Raumtemperatur hydriert. Nachdem 2,2 l Wasserstoff aufgenommen worden sind, filtriert man den Katalysator ab und dampft das Filtrat ein. Zum Rückstand gibt man 800 ml Methylenchlorid und 300 ml Wasser, neutralisiert (pH = 7) unter Umschütteln mit gesättigter Natriumbicarbonatlösung, trennt die Methylenchloridphase ab und extrahiert die wässerige Phase zweimal mit je 100 ml Methylenchlorid. Die Methylenchloridphasen werden

vereinigt, getrocknet und eingedampft, und der Rückstand wird aus Aethanol/Wasser (3:1) umkristallisiert; man erhält 1-(4-Hydroxy-3,5-di-tert.-butylbenzylamino)imidazol vom Smp. 178-180°.

Beispiel 8

1,20 g 1-Aminoimidazol-hydrochlorid werden zusammen mit 2,70 g 4-Hydroxy-3,5-di-tert.-butylbenzoylchlorid auf dem Oelbad (ca. 200-210° Badtemperatur) geschmolzen, wobei eine heftige Gasentwicklung auftritt. Nach 40 Minuten verrührt man den festen Rückstand mit heissem Diisopropyläther und filtriert, nimmt den Filterrückstand in Wasser auf, versetzt bis zur alkalischen Reaktion mit gesättigter Natriumbicarbonatlösung und extrahiert dreimal mit Methylenchlorid. Die vereinten organischen Extrakte werden über wasserfreiem Magnesiumsulfat getrocknet, filtriert und unter vermindertem Druck am Rotationsverdampfer eingedampft. Man nimmt den Rückstand in Essigester auf, erhitzt und fällt das 1-(4-Hydroxy-3,5-di-tert.-butylbenzoylamino)imidazol in der Hitze mit n-Hexan aus. Der Niederschlag wird abfiltriert, einmal mit n-Hexan/Aethylacetat (2:1) gewaschen und bei 50° im Hochvakuum getrocknet; Smp. 245° (Zers.).

Beispiel 9

Aus 1-(4-Hydroxy-3,5-di-tert.-butylbenzoylamino)imidazol kann man in Analogie zu den Angaben in Beispiel 4 1-(4-Hydroxy-3,5-di-tert.-butylbenzylamino)imidazol erhalten.

Beispiel 10

a) Eine Lösung von 120 ml Schwefelkohlenstoff und 60,6 g Dicyclohexylcarbodiimid in 300 ml Tetrahydrofuran wird mit einer Eis/Salz-Mischung auf -8° gekühlt. Hierauf werden 39,9 g Aminoacetaldehyd-diäthylacetal unter Rühren so zugetropft, dass die Temperatur des Reaktionsgemisches -5° nicht übersteigt (ungefähr 1 Stunde). Dann wird das Gemisch langsam unter Rühren auf Raumtemperatur erwärmt (ungefähr 2 Stunden) und über Nacht stehen gelassen. Der entstandene Niederschlag (Dicyclohexylthioharnstoff) wird dann abfiltriert und gründlich mit n-Hexan gewaschen. Das Filtrat wird im Vakuum eingeengt, und der ausgefallene Dicyclohexylthioharnstoff wird wieder abfiltriert und mit n-Hexan gewaschen. Dieser Vorgang wird solange wiederholt, bis beim Einengen kein Dicyclohexylthioharnstoff mehr ausfällt. Zum Schluss wird das Filtrat gänzlich von Lösungsmitteln befreit. Der Rückstand wird bei 11 Torr destilliert (Badtemperatur 130-134°), wobei man 2,2-Diäthoxyäthylisothiocyanat in Form eines farblosen Oels erhält; Sdp. 100°/11 Torr.

b) 2,5 g Hydrazinhydrat (98-100%ig) werden in 10 ml 96%igem Aethanol gelöst. Diese Lösung wird unter Rühren tropfenweise mit 8,75 g 2,2-Diäthoxyäthylisothiocyanat versetzt, und zwar so, dass die Temperatur 40° nicht übersteigt. Nach Beendigung der Zugabe und Abkühlen erstarrt der Kolbeninhalt völlig. Das Aethanol wird im Vakuum bei 30-40° (Badtemperatur) abgedampft. Das als farbloser kristalliner Rückstand verbleibende 4-(2′,2′-Diäthoxyäthyl)thiosemicarbazid (Smp. 92-97°) kann ohne Reinigung weiterverarbeitet werden. Eine aus Wasser umkristallisierte Probe zeigt einen Schmelzpunkt von 95-97°.

c) 2,07 g 4-(2′,2′-Diäthoxyäthyl)thiosemicarbazid werden mit 10 ml 2N Schwefelsäure versetzt und 15 Minuten unter Rückfluss auf dem siedenden Wasserbad erhitzt. Die gelbliche Lösung wird dann bei ca 40° mit 1,06 g Benzaldehyd versetzt und gut gerührt. Nach Abkühlen auf Raumtemperatur wird der entstandene gelbe Niederschlag abfiltriert, mit Wasser gewaschen und getrocknet. Zur Reinigung wird das Rohprodukt in ca. 15 ml heissem Wasser suspendiert und in der Hitze mit soviel Aethanol versetzt, dass vollständige Lösung eintritt. Diese Lösung wird heiss filtriert und anschliessend abgekühlt. Dabei fällt 1-Benzylidenamino-2-mercaptoimidazol in Form gelber Nadeln aus. Eine Probe wird 12 Stunden bei 40° und 10 Torr getrocknet und zeigt dann einen Smp. von 158-161°.

d) 10,16 g 1-Benzylidenamino-2-mercaptoimidazol werden in 30 ml absolutem Aethanol suspendiert und mit einer Lösung von 1,15 g Natrium in 70 ml absolutem Aethanol versetzt. Nach Zugabe von 7,1 g Methyljodid rührt man das Reaktionsgemisch während 3 Stunden bei Raumtemperatur. Zur Aufarbeitung engt man die entstandene gelbe Lösung auf ein Viertel ein und gibt 100 ml Wasser zu. Das resultierende kristalline Rohprodukt wird abfiltriert, dreimal mit Wasser gewaschen und aus Methanol/Wasser umkristallisiert; man erhält 1-Benzylidenamino-2-methylmercaptoimidazol vom Smp. 95-97°.

e) 2,17 g 1-Benzylidenamino-2-methylmercaptoimidazol werden in 50 ml Wasser suspendiert und nach Zugabe von 6 ml 2N Salzsäure einer Wasserdampfdestillation unterzogen. Nach Beendigung der Benzaldehydabspaltung engt man die entstandene Lösung bis auf wenige ml ein, gibt bis zur alkalischen Reaktion 5N Natronlauge zu und extrahiert fünfmal mit je 15 ml Methylenchlorid. Die vereinigten

Methylenchloridphasen werden über Natriumsulfat getrocknet, filtriert und eingedampft. Der kristalline Rückstand wird aus n-Hexan/Essigester umkristallisiert, und man erhält 1-Amino-2-methylmercaptoimidazol in Form farbloser Kristalle vom Smp. 71-73° (Subl. ab ca. 50°).

f) 0,65 g 1-Amino-2-methylmercaptoimidazol werden in 50 ml Methanol gelöst und mit 3 ml 2N Salzsäure versetzt. Nach Zugabe von 1,05 g 4-Hydroxy-3,5-di-tert.-butylbenzaldehyd wird das Reaktionsgemisch über Nacht bei Raumtemperatur gerührt. Anschliessend dampft man das Lösungsmittel im Vakuum ab, suspendiert den Rückstand in 30 ml Wasser und versetzt bis zur alkalischen Reaktion mit gesättigter Natriumcarbonatlösung. Man extrahiert dreimal mit je 20 ml Methylenchlorid, trocknet die vereinigten organischen Phasen über Natriumsulfat, filtriert und dampft ein. Der kristalline Rückstand wird aus Wasser/Methanol umkristallisiert; man erhält 1-(4-Hydroxy-3,5-di-tert.-butylbenzylidenamino)-2-methylmercaptoimidazol vom Smp. 181-183°.

Beispiel 11

1,04 g 1-(4-Hydroxy-3,5-di-tert.-butylbenzylidenamino)-2-methylmercaptoimidazol werden in 20 ml Eisessig gelöst und innerhalb von 30 Minuten portionenweise mit 0,43 g 90%igem Natriumcyanoborhydrid versetzt. Das Reaktionsgemisch wird über Nacht bei Raumtemperatur gerührt, worauf man das Lösungsmittel im Vakuum abdampft und den Rückstand mit 20 ml Wasser aufnimmt. Man gibt bis zur alkalischen Reaktion gesättigte Natriumcarbonatlösung zu und extrahiert dreimal mit je 15 ml Methylenchlorid. Die vereinigten organischen Phasen werden über Natriumsulfat getrocknet, filtriert und eingedampft. Der Rückstand wird aus Methanol/Wasser umkristallisiert, und man erhält 1-(4-Hydroxy-3,5-di-tert.-butylbenzylamino) -2-methylmercaptoimidazol vom Smp. 110-113°.

Beispiel 12

Variante a) : 200 mg 1-(4-Hydroxy-3,5-di-tert.-butylbenzylamino)-2-methylmercaptoimidazol werden zusammen mit 0,41 g Nickel(II)chlorid•$6H_2O$ in 8 ml Methanol gelöst. Man kühlt diese Lösung im Eisbad und gibt innerhalb 1 Stunde portionenweise 0,7 g Natriumborhydrid zu. Nach beendeter Zugabe entfernt man das Eisbad und rührt das schwarze Reaktionsgemisch noch weitere 4 Stunden bei Raumtemperatur. Zur Aufarbeitung kühlt man wiederum ab, gibt soviel 2N Salzsäure zu, dass das Reaktionsgemisch sauer reagiert, rührt einige Minuten und versetzt bis zur alkalischen Reaktion mit konz. Ammoniak. Anschliessend filtriert man durch ein Bett von Kieselgur und wäscht den Filterkuchen fünfmal mit Methylenchlorid. Die organische Phase des Filtrats wird abgetrennt, zweimal mit Wasser gewaschen, über Natriumsulfat getrocknet und eingedampft. Der kristalline Rückstand wird aus Methanol/Wasser umgefällt, und man erhält 1-(4-Hydroxy-3,5-di-tert.-butylbenzylamino)imidazol in Form farbloser Kristalle vom Smp. 171-174°.

Variante b): 600 mg 1-(4-Hydroxy-3,5-di-tert.-butylbenzylamino)-2-methylmercaptoimidazol, gelöst in 20 ml Aethanol, werden unter Rühren mit der 5-fachen Gewichtsmenge an äthanolfeuchtem Raney-Nickel W2 während 2 Stunden auf 60-70° erwärmt. Das Nickel wird dann abfiltriert und zweimal mit je 10 ml Aethanol ausgekocht. Die vereinigten alkoholischen Filtrate werden eingedampft. Als Rückstand erhält man 1-(4-Hydroxy-3,5-di-tert.-butylbenzylamino)imidazol in Form farbloser Kristalle vom Smp. 171-174°.

Beispiel 13

300 mg 1-(4-Hydroxy-3,5-di-tert.-butylbenzylamino)imidazol werden zusammen mit 10 Tropfen Eisessig in 25 ml Benzol am Wasserbad auf ca. 60-70° erwärmt. Nach Zutropfen einer Lösung von 250 mg Dicyanodichlorbenzochinon in 10 ml Benzol erwärmt man weitere 15 Minuten. Anschliessend kühlt man das Reaktionsgemisch im Eisbad und filtriert den ausgefallenen Festkörper ab. Das Filtrat wird mit 10%iger Natriumcarbonatlösung geschüttelt. Nach Abtrennen der organischen Phase wird die Wasserphase einmal mit Benzol extrahiert. Die vereinigten organischen Phasen werden mit wasserfreiem Magnesiumsulfat getrocknet, filtriert und unter vermindertem Druck eingedampft. Der kristalline Rückstand wird aus Essigester/n-Hexan umkristallisiert, und man erhält 1-(4-Hydroxy-3,5-di-tert.-butylbenzylidenamino)imidazol vom Smp. 173°.

Beispiel 14

a) 15 ml Essigsäureanhydrid und 7,5 ml Ameisensäure (98-100%) werden bei 0° vorsichtig gemischt und anschliessend 1 Stunde am Wasserbad auf 50° erwärmt. Das gebildete Mischanhydrid wird mit 25

ml abs. Tetrahydrofuran verdünnt, und in die erhaltene Lösung werden 4,52 g 1-(4-Hydroxy-3,5-di-tert.-butylbenzylamino)imidazol in kleinen Portionen eingetragen. Man lässt das Reaktionsgemisch 1 Stunde bei Raumtemperatur stehen, dampft es unter vermindertem Druck am Rotationsverdampfer ein, versetzt den Rückstand mit wenig eiskaltem Wasser und gibt bis zur schwach alkalischen Reaktion gesättigte Natriumbicarbonatlösung zu. Nach dreimaliger Extraktion mit Methylenchlorid werden die vereinigten organischen Extrakte einmal mit Wasser und einmal mit gesättigter Kochsalzlösung gewaschen, über wasserfreiem Magnesiumsulfat getrocknet, filtriert und eingedampft. Der Rückstand wird aus Essigester/n-Hexan umkristallisiert, und man erhält 1-[N-Formyl-N-(4-hydroxy-3,5-di -tert.-butylbenzyl)-amino]imidazolvom Smp. 167°.

b) 3,30 g 1-[N-Formyl-N-(4-hydroxy-3,5-di -tert.-butylbenzyl)amino]imidazol werden in 100 ml absolutem Tetrahydrofuran gelöst; zu dieser Lösung spritzt man bei Raumtemperatur 20 ml einer 2 molaren Lösung von Boran-Dimethylsulfid-Komplex [$BH_3 \cdot S(CH_3)_2$] in Toluol. Das Reaktionsgemisch wird anschliessend 2 Stunden zu gelindem Rückfluss erhitzt, auf 0° gekühlt, mit 20 ml absolutem Methanol versetzt und weitere 30 Minuten erhitzt. Das Lösungsmittel wird unter vermindertem Druck am Rotationsverdampfer vollständig abgedampft; der verbleibende kristalline Festkörper wird mit n-Hexan verrührt, abfiltriert und während kurzer Zeit trockengesaugt. Hierauf suspendiert man den erhaltenen Festkörper in 50 ml absolutem Methanol und leitet unter Rühren bis zur Sättigung vorsichtig Chlorwasserstoff ein, wobei unter starker Erwärmung eine klare Lösung entsteht. Diese Lösung wird 1 Stunde zu gelindem Rückfluss erhitzt und dann unter vermindertem Druck am Rotationsverdampfer eingedampft. Der Rückstand wird mit 25 ml eiskaltem Wasser versetzt, worauf man bis zur schwach alkalischen Reaktion gesättigte Natriumbicarbonatlösung zugibt und dreimal mit je 25 ml Methylenchlorid extrahiert. Die vereinigten Methylenchloridextrakte werden einmal mit wenig Wasser und anschliessend mit gesättigter Kochsalzlösung gewaschen, über wasserfreiem Magnesiumsulfat getrocknet, filtriert und eingedampft. Der Rückstand wird aus Essigester/n-Hexan umkristallisiert, und man erhält 1-[N-(4-Hydroxy-3,5-di-tert.-butylbenzyl) -N-methylamino]imidazol vom Smp. 139°.

Beispiel 15

a) 0,6 g 1-(4-Hydroxy-3,5-di-tert.-butylbenzylamino)imidazol werden in 5,3 ml Ameisensäure gelöst, worauf man unter Eiskühlung 1,9 ml Essigsäureanhydrid zugibt und das Gemisch über Nacht stehen lässt. Hierauf dampft man im Vakuum ein, gibt Wasser zu und dampft erneut ein. Zum kristallinen Rückstand gibt man Wasser und Methylenchlorid und neutralisiert (pH = 7) mit Natriumbicarbonatlösung. Die organische Phase wird abgetrennt, getrocknet und im Vakuum eingedampft. Man rührt den Rückstand mit Aether auf und filtriert; das auf dem Filter zurückbleibende 1-[N-Formyl-N-(4-hydroxy-3,5-di -tert.-butylbenzyl)amino]imidazol zeigt einen Smp. von 169-170°.

b) Das 1-[N-Formyl-N-(4-hydroxy-3,5-di -tert.-butylbenzyl)amino]imidazol kann gemäss den Angaben in Beispiel 14b) in 1-[N-(4-Hydroxy-3,5-di-tert.-butylbenzyl) -N-methylamino]imidazol übergeführt werden.

Beispiel 16

a) 680 mg Imidazol werden in einem Gemisch aus 4 ml absolutem N-Methyl-2-pyrrolidon und 1 ml absolutem Tetrahydrofuran suspendiert. Die Suspension wird auf -40° gekühlt. Unter Rühren wird innterhalb von 30 Minuten eine Lösung von 4,8 g 0-(2,4-Dinitrophenyl)hydroxylamin in 8 ml absolutem N-Methyl-2-pyrrolidon langsam zugetropft. Man lässt bei 20° während 18 Stunden stehen, versetzt das Reaktionsgemisch mit 25 ml Wasser und 12 ml 2N Salzsäure und schüttelt fünfmal mit je 20 ml Aether aus. Die saure Lösung wird mit Aktivkohle während 15 Minuten gerührt und darauf filtriert. Nach Zugabe von 3 ml Benzaldeyd, 5 ml 2N Salzsäure und 30 ml Aether wird drei Stunden unter Eiskühlung gerührt. Man filtriert den ausgefallenen Niederschlag ab und wäscht ihn zweimal mit Wasser und einmal mit Aether. Das so erhaltene Rohprodukt fällt man aus Methanol/Aether um. Man erhält 1,3-Bis-(benzylidenamino)imidazoliumchlorid vom Schmelzpunkt 193-196°.

b) 3,11 g 1,3-Bis(benzylidenamino)imidazoliumchlorid, 0,32 g Schwefel und 1,01 g Triäthylamin werden in 20 ml absolutem Pyridin suspendiert, worauf man während 15 Minuten bei Raumtemperatur rührt. Anschliessend erhitzt man das Reaktionsgemisch eine halbe Stunde lang unter Rückfluss zum Sieden. Nach Abkühlen auf Raumtemperatur versetzt man mit 100 ml Wasser, nutscht den entstandenen Niederschlag ab und wäscht dreimal mit Wasser und dreimal mit Aethanol. Man erhält hellgelbes 1,3-Bis(benzylidenamino)-1,3-dihydro-2H-imidazol-2-thion vom Schmelzpunkt 215-218°.

c) Man versetzt 3,06 g 1,3-Bis(benzylidenamino)-1,3-dihydro-2H-imidazol-2-thion in 100 ml absolutem Methylenchlorid mit 1,47 g Trimethyloxoniumtetrafluoroborat, rührt während 3 Stunden bei Raumtemperatur und nutscht den entstandenen Niederschlag ab. Aus dem Filtrat wird durch Zugabe von Diäthyläther eine weitere Portion des gewünschten Produktes ausgefällt, die zuammen mit der ersten Portion einmal mit Wasser, einmal mit kaltem Methanol und dreimal mit Diäthyläther gewaschen und anschliessend aus Acetonitril/Diäthyläther umgefällt wird. Man erhält farbloses 1,3-Bis(benzylidenamino)-2 -(methylthio)imidazoliumtetrafluoroborat vom Schmelzpunkt 215-220° (Zersetzung).

d) 4,1 g 1,3-Bis(benzylidenamino)-2-(methylthio)imidazolium-tetrafluoroborat in 25 ml absolutem Acetonitril werden unter Argonatmosphäre mit 1,45 g absolutem Morpholin versetzt, wobei sofort Methylmercaptanentwicklung einsetzt und Lösung eintritt. Man lässt über Nacht bis zur Beendigung der Methylmercaptanabspaltung bei Raumtemperatur rühren, filtriert den entstandenen farblosen Niederschlag ab, versetzt das Filtrat mit 30 ml Diäthyläther und filtriert erneut ab. Die beiden Filterrückstände werden vereinigt und aus Acetonitril/Aether umgefällt; man erhält 1,3-Bis(benzylidenamino)-2-morpholinoimidazolium -tetrafluoroborat in Form farbloser Kristalle vom Smp. 244-247° (Zers.).

e) 4,47 g 1,3-Bis(benzylidenamino)-2-morpholinoimidazolium-tetrafluoroboratwerden in 50 ml Methanol suspendiert. Unter Kühlen im Eisbad tropft man innerhalb von 15 Minuten eine Lösung von 1,3 g Kaliumcyanid in 20 ml Wasser zu, wobei eine gelbe Lösung entsteht. Man rührt noch 15 Minuten bei Raumtemperatur nach, gibt 150 ml Wasser zu und extrahiert dreimal mit je 30 ml Methylenchlorid. Die vereinigten Methylenchloridextrakte werden über Natriumsulfat getrocknet, filtriert und im Vakuum eingedampft. Das als Rückstand verbleibende gelbe Oel wird mit 70 ml eisgekühltem Wasser versetzt, worauf man mit 2N Salzsäure ansäuert (pH ca. 1) und anschliessend dreimal mit Diäthyläther extrahiert. Die farblose, saure wässrige Phase wird bis zur alkalischen Reaktion (pH ca. 8-9) mit 2N Natronlauge versetzt. Der entstehende hellgelbe Niederschlag wird abfiltriert und aus Methanol/Wasser umgefällt; man erhält 1-Benzylidenamino-2-morpholinoimidazol vom Smp. 59-61°.

f) 3,85 g 1-Benzylidenamino-2-morpholinoimidazol werden in 20 ml 1N Salzsäure gelöst und solange einer Wasserdampfdestillation unterzogen, bis kein Benzaldehyd mehr übergeht. Die zurückbleibende farblose saure Lösung wird auf ca. 10 ml eingeengt, unter Kühlen bis zur alkalischen Reaktion mit 5N Natronlauge versetzt und dann mit Kochsalz gesättigt. Man extrahiert fünfmal mit je 25 ml Methylenchlorid, trocknet die vereinigten organischen Phasen über Natriumsulfat, filtriert und dampft das Lösungsmittel im Vakuum ab. Das als Rückstand verbleibende farblose, kristalline 1-Amino-1-morpholinoimidazol ist für die Weiterverarbeitung bereits rein genug. Eine durch Umkristallisation aus n-Hexan/Essigester oder durch Sublimation gereinigte Probe zeigt einen Smp. von 150-151° (ab 120° Sublimation).

g) 1,18 g 1-Amino-2-morpholinoimidazol werden in 50 ml Aethanol gelöst und mit 3,5 ml 2N Salzsäure versetzt. Nach Zugabe von 1,65 g 4-Hydroxy-3,5-di-tert.-butylbenzaldehyd wird das Reaktionsgemisch über Nacht bei Raumtemperatur gerührt. Anschliessend dampft man das Lösungsmittel im Vakuum ab, versetzt den Rückstand mit 20 ml Wasser und gibt bis zur alkalischen Reaktion gesättigte Natriumcarbonatlösung zu. Das ausfallende, farblose Produkt wird abfiltriert und säulenchromatographisch gereinigt (Laufmittel Methylenchlorid/Methanol 10%). Durch Umkristallisation aus Wasser/Methanol erhält man reines 1-(4-Hydroxy-3,5-di-tert.-butylbenzylidenamino) -2-morpholinoimidazol vom Smp. 185-187°.

## Beispiel 17

1,92 g 1-(4-Hydroxy-3,5-di-tert.-butylbenzylidenamino)-2-morpholinoimidazol werden in 30 ml Eisessig gelöst und innerhalb von 30 Minuten portionenweise mit 0,7 g 90%igem Natriumcyanoborhydrid versetzt. Das Reaktionsgemisch wird über Nacht gerührt, worauf man das Lösungsmittel im Vakuum abdampft und den Rückstand mit 20 ml Wasser versetzt. Man gibt bis zur alkalischen Reaktion gesättigte Natriumcarbonatlösung zu und extrahiert dreimal mit je 20 ml Methylenchlorid. Die organischen Phasen werden vereinigt, über Natriumsulfat getrocknet, filtriert und eingedampft. Der Rückstand wird kurz mit 20 ml n-Hexan aufgekocht. Nach dem Abkühlen wird das kristalline farblose Produkt abfiltriert und aus Methanol/Wasser umkristallisiert; man erhält 1-(4-Hydroxy-3,5-di-tert.-butylbenzylamino) -2-morpholinoimidazol vom Smp. 161-163°.

## Beispiel 18

a) 12,5 g 2-Isopropylimidazol werden in 35 ml Wasser suspendiert und innerhalb von 30 Minuten unter Rühren mit einer bei 0° hergestellten Lösung aus 12,5 g Hydroxylamino-O-sulfonsäure und 9,25 g Natriumbicarbonat in 75 ml Eiswasser versetzt. Nach 20-stündigem Rühren bei Raumtemperatur säuert man mit 32,5 ml 2N Salzsäure an, fügt eine Lösung von 7,75 g Benzaldehyd in 25 ml Aether zu und rührt während 6 Stunden bei Raumtemperatur. Nach Abdampfen des Aethers wird gründlich mit Methylenchlorid extrahiert; der Auszug wird über Natriumsulfat getrocknet und eingedampft. Der Rückstand wird durch Zugabe von Methylenchlorid kristallisiert. Nach Umkristallisieren aus Methylenchlorid erhält man 1-Benzylidenamino-2-isopropylimidazolium-benzaldoxim-O-sulfonat vom Smp. 147-148°.

5,3 g des obigen Salzes werden in 100 ml Methylenchlorid suspendiert, worauf 70 ml gesättigte Natriumhydrogencarbonatlösung zugegeben werden. Das Produkt wird mit Methylenchlorid extrahiert und aus Aether/Petroläther kristallisiert. Man erhält 1-Benzylidenamino-2-isopropylimidazol vom Smp. 65-66°.

b) Ein Gemisch von 1 g 1-Benzylidenamino-2-isopropylimidazol und 50 ml 1N Salzsäure wird so lange einer Wasserdampfdestillation unterworfen, bis kein Benzaldehyd mehr übergeht. Die zurückbleibende Lösung wird im Vakuum eingedampft. Zum Rückstand gibt man Methanol und Benzol, dampft ein, gibt nochmals Methanol und Benzol zu und dampft erneut ein. Man erhält rohes 1-Amino-2-isopropylimidazol-hydrochlorid.

c) Man löst 0,65 g des obigen rohen 1-Amino-2-isopropylimidazol-hydrochlorids in 30 ml Aethanol, gibt 0,94 g 4-Hydroxy-3,5-di-tert.-butylbenzaldehyd zu und rührt das Gemisch während 2 Stunden bei Raumtemperatur. Die entstandene Lösung wird im Vakuum eingedampft, worauf man den Rückstand zwischen Methylenchlorid und Wasser verteilt, die wässrige Phase bis zur neutralen Reaktion (pH = 7) mit Natriumbicarbonatlösung versetzt und die Methylenchloridphase abtrennt, trocknet, filtriert und eindampft. Der Rückstand wird mit Aether aufgerührt, worauf man den Festkörper abfiltriert und trocknet; man erhält 1-(4-Hydroxy-3,5-di-tert.-butylbenzylidenamino) -2-isopropylimidazol vom Smp. 159-160°.

Beispiel 19

a) Man löst 71 g Hydroxylamin-O-sulfonsäure in 420 ml Eiswasser und neutralisiert durch Zugabe von 54 g Natriumbicarbonat. Die erhaltene Lösung wird innerhalb von 30 Minuten zu einer Lösung von 25,8 g 4-(5)-Methylimidazol in 320 ml Wasser getropft, wobei der pH durch ständige Zugabe (Autotitrator) von 1N Natronlauge stets bei 9,5 gehalten wird. Man rührt über Nacht bei Raumtemperatur, gibt dann bis zur sauren Reaktion (pH = 1,5) konz. Salzsäure zu, versetzt mit einer Lösung von 64 ml Benzaldehyd in 200 ml Aether und rührt während 6 Stunden bei Raumtemperatur, wobei bereits nach ca. 30 Minuten Kristallisation einsetzt.

Der ausgefallene Festkörper wird abfiltriert, mit Wasser und Aether gewaschen und bei 60° getrocknet; man erhält 1,3-Bis(benzylidenamino)-4(5)-methylimidazoliumchlorid vom Smp. 137-139° (Zers.). Das Filtrat wird durch Zugabe von 2N Natronlauge auf pH 6 gestellt, worauf das ausgefallene Oel mit Essigester extrahiert wird. Die Essigesterphase wird abgetrennt, mit Wasser gewaschen, getrocknet und im Vakuum eingedampft. Der ölige Rückstand wird in 100 ml heissem Isopropyläther gelöst. Die beim Abkühlen ausgefallenen Kristalle werden abfiltriert und zuerst mit Isopropyläther und dann mit Petroläther gewaschen; man erhält 1-Benzylidenamino-4-methylimidazol vom Smp. 91-93°.

b) 5,7 g 1-Benzylidenamino-4-methylimidazol in 50 ml Wasser und 30 ml 2N Salzsäure werden einer Wasserdampfdestillation unterzogen, bis kein Benzaldehyd mehr übergeht. Die zurückbleibende Lösung wird über Kohle filtriert und im Vakuum eingedampft. Man versetzt den Rückstand mit Methanol und Toluol, dampft ein, gibt nochmals Methanol und Toluol zu und dampft erneut ein. Man nimmt den Rückstand in Aethanol auf und führt durch Zugabe von Aether Kristallisation herbei. Der ausgefallene Festkörper wird abfiltriert, mit Aether und Hexan gewaschen und bei 60° im Vakuum getrocknet; man erhält 1-Amino-4-methylimidazol-hydrochlorid vom Smp. 114-155°.

c) Man löst 3,5 g 1-Amino-4-methylimidazol-hydrochlorid in 200 ml Aethanol, gibt 6,2 g 4-Hydroxy-3,5-di-tert.-butylbenzaldehyd zu und rührt über Nacht bei Raumtemperatur. Dann dampft man die erhaltene Lösung im Vakuum ein, versetzt den Rückstand mit 150 ml Methylenchlorid und 150 ml Wasser, rührt und gibt bis zur neutralen Reaktion (pH = 7) gesättigte Natriumbicarbonatlösung zu. Man trennt die Methylenchloridphase ab und extrahiert die wässerige Phase mit 50 ml Methylenchlorid, worauf die Methylenchloridphasen vereinigt, einmal mit 10%iger Kochsalzlösung gewaschen, getrocknet und eingedampft werden. Man verrührt den Rückstand mit kaltem Aether, worauf der Festkörper abfiltriert, mit Aether und n-Hexan gewaschen und bei 70° im Vakuum getrocknet wird; man erhält 1-(4-Hydroxy-3,5-di-tert.-butylbenzylidenamino)-4-methylimidazol vom Smp. 150-151°.

Beispiel 20

6,1 g 1-(4-Hydroxy-3,5-di-tert.-butylbenzylidenamino)-4-methylimidazol werden in 100 ml Methanol, 21 ml 1N Salzsäure und 30 ml Wasser in Gegenwart von Palladium-Kohle bei Normaldruck und Raumtemperatur hydriert. Nachdem 500 ml Wasserstoff aufgenommen worden sind, filtriert man, dampft das Filtrat auf ein Volumen von 50 ml ein, gibt 50 ml Wasser zu und versetzt bis zur neutralen Reaktion (pH = 7) mit gesättigter Natriumbicarbonatlösung. Die ausgefallenen Kristalle werden abfiltriert und mit Methylenchlorid

aufgenommen, worauf die Methylenchloridlösung einmal mit Wasser gewaschen, getrocknet und eingedampft wird. Der Rückstand wird mit kaltem Aether verrührt, wonach der Festkörper abfiltriert, mit Aether und n-Hexan gewaschen und bei 60° im Vakuum getrocknet wird. Man erhält 1-(4-Hydroxy-3,5-di-tert.-butylbenzylamino)-4-methylimidazol vom Smp. 150-151°.

Beispiel 21

a) 25 g 2-Propylimidazol werden in 70 ml Wasser suspendiert und innerhalb von 20 Minuten unter Rühren mit einer bei 0° hergestellten Lösung aus 25 g Hydroxylamin-O-sulfonsäure und 18,5 g Natriumbicarbonat in 150 ml Wasser versetzt. Nach 20-stündigem Rühren bei Raumtemperatur säuert man mit 65 ml 2N Salzsäure an, fügt eine Lösung von 15,5 g Benzaldehyd in 50 ml Aether hinzu und rührt 6 Stunden bei Raumtemperatur. Die gebildeten weissen Kristalle werden abfiltriert; das Filtrat wird wie weiter unten beschrieben behandelt.

Die Kristalle werden in Methylenchlorid gelöst. Die Lösung wird über Natriumsulfat getrocknet, filtriert und eingeengt; der Rückstand wird durch Zugabe von Aether kristallisiert. Nach Umkristallisation aus Aethanol erhält man 1,3-Bis(benzylidenamino)-2-propylimidazolium-benzaldoxim-O-sulfonatvom Smp. 143-145°.

Das obige Filtrat wird mit Aether gewaschen, mit 60 ml 3N Natronlauge versetzt und mit Methylenchlorid extrahiert. Nach Trocknen des Auszuges über Natriumsulfat, Filtration und Abdampfen des Methylenchlorids erhält man ein rötliches Oel, aus welchem man durch Chromatographie an Kieselgel (Korngrösse 0,063-0,2 mm) mittels Methylenchlorid/Methanol (99:1%) 1-Benzylidenamino-2-propylimidazol vom Smp. 61-62° erhält.

b) Eine Suspension von 14 g 1-Benzylidenamino-2-propylimidazol in 113 ml Wasser und 79 ml 2N Salzsäure wird so lange einer Wasserdampfdestillation unterzogen, bis kein Benzaldehyd mehr übergeht. Die zurückbleibende, leicht trübe Lösung wird abgekühlt und im Vakuum eingedampft. Zum öligen Rückstand gibt man Methanol und Benzol, dampft ein, gibt 30 ml Aethanol zu und versetzt die erhaltene Lösung bis zur beginnenden Trübung mit Aether. Nach Abkühlen werden die ausgefallenen Kristalle abfiltriert, mit Aether und n-Hexan gewaschen und bei 60° im Vakuum getrocknet. Man erhält 1-Amino-2-propylimidazol-hydrochlorid vom Smp. 108-109°.

c) 6,47 g 1-Amino-2-propylimidazol-hydrochlorid werden in 300 ml Aethanol gelöst, worauf man 3,5 g 4-Hydroxy-3,5-di-tert.-butylbenzaldehyd zugibt, 3 Stunden bei Raumtemperatur rührt und die erhaltene Lösung eindampft. Der Rückstand wird mit 150 ml Wasser und 150 ml Methylenchlorid versetzt, worauf man unter Rühren bis zur neutralen Reaktion (pH = 7) gesättigte Natriumbicarbonatlösung zugibt. Die Methylenchloridphase wird abgetrennt, und die wässerige Phase wird einmal mit 50 ml Methylenchlorid extrahiert, worauf die Methylenchloridlösungen vereinigt, getrocknet und eingedampft werden. Der Rückstand wird mit Aether verrührt, worauf der Festkörper abfiltriert und bei 60° im Vakuum getrocknet wird. Man erhält 1-(4-Hydroxy-3,5-di-tert.-butylbenzylidenamino) -2-propylimidazol vom Smp. 160-161°.

Beispiel 22

9,9 g 1-(4-Hydroxy-3,5-di-tert.-butylbenzylidenamino)-2-propylimidazol werden in 200 ml Methanol und 29 ml 1N Salzsäure in Gegenwart von 1 g eines Palladiumkatalysators (5% auf Kohle) bei Normaldruck und Raumtemperatur hydriert. Nachdem 700 ml Wasserstoff aufgenommen worden sind, filtriert man vom Katalysator ab und dampft das Filtrat ein. Der Rückstand wird mit 100 ml Wasser aufgenommen, worauf man bis zur neutralen Reaktion (pH = 7) gesättigte Natriumbicarbonatlösung zugibt. Die ausgefallenen Kristalle werden abfiltriert und in 300 ml Methylenchlorid aufgenommen, worauf die Methylenchloridlösung einmal mit Wasser gewaschen, getrocknet und eingedampft wird. Der Rückstand wird in 50 ml heissem Isopropyläther gelöst. Die beim Abkühlen ausgefallenen Kristalle werden abfiltriert, mit Isopropyläther und n-Hexan gewaschen und bei 60° im Vakuum getrocknet. Man erhält 1-(4-Hydroxy-3,5-di-tert.-butylbenzylamino)-2-propylimidazol vom Smp. 122-123°.

Beispiel A

Es werden in üblicher Weise Suppositorien folgender Zusammensetzung hergestellt:

| | |
|---|---|
| 1-(4-Hydroxy-3,5-di-tert.-butylbenzylamino)imidazol | 0,025 g |
| Hydriertes Kokosnussöl | 1,230 g |
| Carnaubawachs | 0,045 g |
| Gesamtgewicht | 1,300 g |

Beispiel B

Es werden in üblicher Weise Tabletten folgender Zusammensetzung hergestellt:

| | Pro Tablette |
|---|---|
| 1-(4-Hydroxy-3,5-di-tert.-butylbenzylamino)imidazol | 25,00 mg |
| Lactose | 64,50 mg |
| Maisstärke | 10,00 mg |
| Magnesiumstearat | 0,50 mg |
| Gesamtgewicht | 100,00 mg |

Beispiel C

Es werden in üblicher Weise Kapseln folgender Zusammensetzung hergestellt:

| | Pro Kapsel |
|---|---|
| 1-(4-Hydroxy-3,5-di-tert.-butylbenzylamino)imidazol | 50 mg |
| Lactose | 125 mg |
| Maisstärke | 30 mg |
| Talk | 5 mg |
| Gesamtgewicht der Kapselfüllung | 210 mg |

## Patentansprüche

**Patentansprüche für folgende Vertragsstaaten : AT, BE, CH, DE, FR, GB, IT, LI, LU, NL, SE**

**1.** Imidazolderivate der allgemeinen Formel

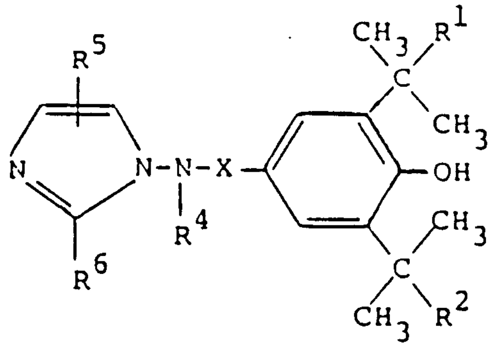

I

worin $R^1$ und $R^2$ je $(C_1\text{-}C_7)$-Alkyl, X einen Rest der Formel

$-CH(R^3)-$ oder $-CO-$ ;

(a) (b)

$R^3$ Wasserstoff und $R^4$ Wasserstoff oder ($C_1$-$C_7$) Alkyl oder $R^3$ und $R^4$ zusammen eine zusätzliche Kohlenstoff-Stickstoff-Bindung; $R^5$ Wasserstoff oder ($C_1$-$C_7$)-Alkyl; R Wasserstoff, ($C_1$-$C_7$)-Alkyl, ($C_1$-$C_7$)-Alkylthio oder einen Rest der Formel $-NR^7R^8$; und $R^7$ und $R^8$ je ($C_1$-$C_7$)-Alkyl oder zusammen mit dem Stickstoffatom einen 5- oder 6-gliedrigen gesättigten Heterocyclus bedeuten;
und pharmazeutisch annehmbare Säureadditionssalze davon.

**2.** Verbindungen nach Anspruch 1, worin $R^1$ und R je Methyl bedeuten.

**3.** Verbindungen nach Anspruch 1 oder 2, worin $R^5$ Wasserstoff oder Methyl in 4-Stellung bedeutet.

**4.** Verbindungen nach einem der Ansprüche 1 bis 3, worin $R^6$ Wasserstoff, Methyl, n-Propyl, Isopropyl, Methylmercapto oder Morpholino bedeutet.

**5.** Verbindung nach einem der Ansprüche 1 bis 4, worin $R^3$ Wasserstoff und $R^4$ Wasserstoff oder Methyl oder $R^3$ und $R^4$ zusammen eine zusätzliche Kohlenstoff-Stickstoff -Bindung bedeuten.

**6.** 1-(4-Hydroxy-3,5-di-tert.-butylbenzylamino)imidazol.

**7.** 1-(4-Hydroxy-3,5-di-tert.-butylbenzylidenamino)-2-methylimidazol.

**8.** 1-(4-Hydroxy-3,5-di-tert.-butylbenzylidenamino)imidazol.

**9.** 1-(4-Hydroxy-3,5-di-tert.-butylbenzylamino)-2-methylimidazol.

**10.** 1-(4-Hydroxy-3,5-di-tert.-butylbenzylamino)-2-propylimidazol.

**11.** 1-(4-Hydroxy-3,5-di-tert.-butylbenzylamino)-2-methylmercaptoimidazol.

**12.** 1-(4-Hydroxy-3,5-di-tert.-butylbenzoylamino)-2-methylimidazol;
1-(4-Hydroxy-3,5-di-tert.-butylbenzoylamino)imidazol;
1-(4-Hydroxy-3,5-di-tert.-butylbenzylidenamino)-2-morpholinoimidazol;
1-(4-Hydroxy-3,5-di-tert.-butylbenzylamino)-2-morpholinoimidazol;
1-(4-Hydroxy-3,5-di-tert.-butylbenzylidenamino)-2-isopropylimidazol;
1-(4-Hydroxy-3,5-di-tert.-butylbenzylidenamino)-4-methylimidazol;
1-(4-Hydroxy-3,5-di-tert.-butylbenzylamino)-4-methylimidazol;
1-(4-Hydroxy-3,5-di-tert.-butylbenzylidenamino)-2-propylimidazol; und
1-[N-(4-Hydroxy-3,5-di-tert.-butylbenzyl)-N-methylamino]imidazol.

**13.** Verbindungen gemäss einem der Ansprüche 1 bis 12 zur Anwendung als therapeutische Wirkstoffe.

**14.** Verbindungen gemäss einem der Ansprüche 1 bis 12 zur Anwendung als entzündungshemmende und oedemhemmende Wirkstoffe.

**15.** Verfahren zur Herstellung von Verbindungen der in Anspruch 1 definierten Formel I und von pharmazeutisch annehmbaren Säureadditionssalzen davon, dadurch gekennzeichnet, dass man
a) zur Herstellung einer Verbindung der Formel I, worin X $-CH(R^3)$ und $R^3$ und $R^4$ zusammen eine zusätzliche Kohlenstoff-Stickstoff-Bindung bedeuten,
eine Verbindung der allgemeinen Formel

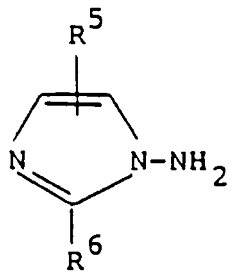

II

worin $R^5$ und $R^6$ die in Anspruch 1 angegebene Bedeutung besitzen, mit einem Aldehyd der allgemeinen Formel

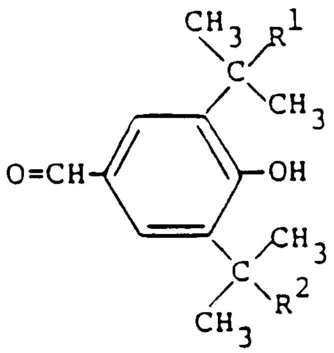

III

worin $R^1$ und $R^2$ die in Anspruch 1 angegebene Bedeutung besitzen, kondensiert; oder

b) zur Herstellung einer Verbindung der Formel I, worin X -CO- und $R^4$ Wasserstoff oder ($C_1$-$C_7$)-Alkyl bedeuten, eine Verbindung der allgemeinen Formel

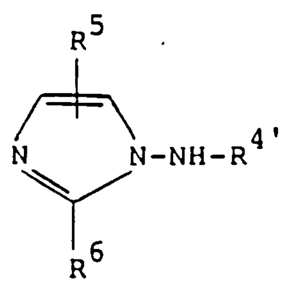

IV

worin $R^5$ und $R^6$ die in Anspruch 1 angegebene Bedeutung besitzen und $R^{4'}$ Wasserstoff oder ($C_1$-$C_7$)-Alkyl bedeutet, mit einem reaktionsfähigen Derivat einer Carbonsäure der allgemeinen Formel

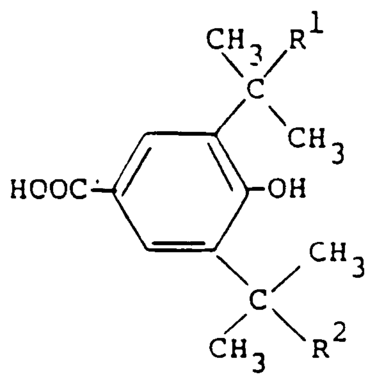

V

worin $R^1$ und $R^2$ die in Anspruch 1 angegebene Bedeutung besitzen, acyliert; oder

c) zur Herstellung einer Verbindung der Formel I, worin X $-CH_2$ und $R^4$ Wasserstoff bedeuten, eine Verbindung der allgemeinen Formel

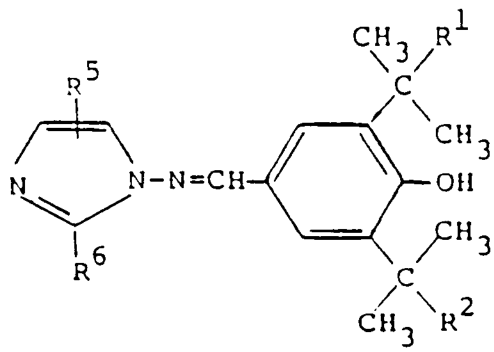

Ia

worin $R^1$, $R^2$, $R^5$ und $R^6$ die in Anspruch 1 angegebene Bedeutung besitzen, reduziert; oder

d) zur Herstellung einer Verbindung der Formel I, worin X $-CH_2$-und $R^4$ Wasserstoff oder $(C_1-C_7)$-Alkyl bedeuten, eine Verbindung der allgemeinen Formel

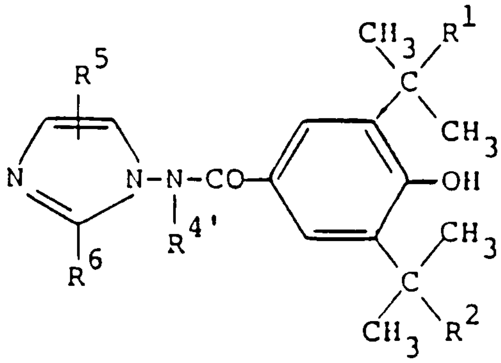

Ib

worin $R^1$, $R^2$, $R^5$ und $R^6$ die in Anspruch 1 angegebene und $R^{4'}$ obige Bedeutung besitzen, reduziert; oder

e) zur Herstellung einer Verbindung der Formel I, worin X $-CH_2$-und $R^4$ $(C_1-C_7)$-Alkyl bedeuten, eine Verbindung der allgemeinen Formel

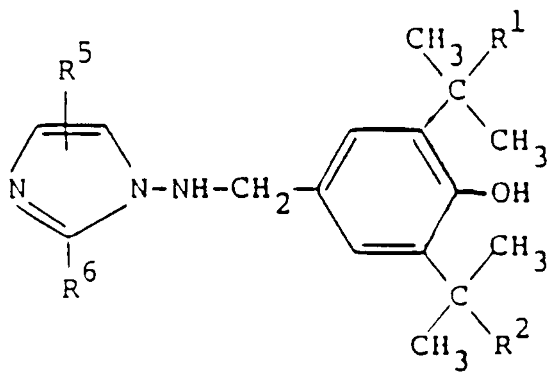

Ic

worin $R^1$, $R^2$, $R^5$ und $R^6$ die in Anspruch 1 angegebene Bedeutung besitzen, entsprechend N-alkyliert; oder

f) zur Herstellung einer Verbindung der Formel I, worin X -CH($R^3$)- und $R^3$ und $R^4$ zusammen eine zusätzliche Kohlenstoff-Stickstoff-Bindung bedeuten, eine Verbindung der obigen Formel Ic dehydriert; oder

g) zur Herstellung einer Verbindung der Formel I, worin X -CO-oder -$CH_2$-, $R^4$ Wasserstoff oder ($C_1$-$C_7$)-Alkyl und $R^6$ Wasserstoff bedeuten, von einer Verbindung der allgemeinen Formel

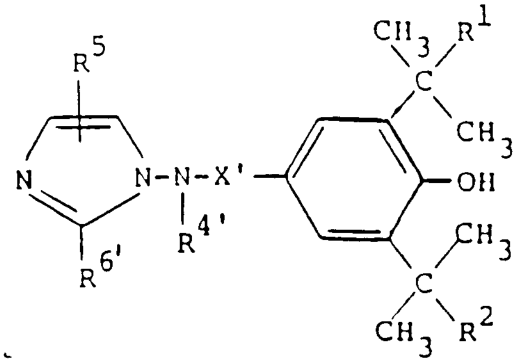

Id

worin $R^1$, $R^2$ und $R^5$ die in Anspruch 1 angegebene und $R^{4'}$ obige Bedeutung besitzen und X' eine Methylen- oder Carbonylgruppe und $R^{6'}$ ($C_1$-$C_7$)-Alkylthio bedeuten, die Alkylthiogruppe entfernt; oder

h) eine Verbindung der Formel I in ein pharmazeutisch annehmbares Säureadditionssalz überführt.

**16.** Arzneimittel, enthaltend eine Verbindung gemäss einem der Ansprüche 1 bis 12 und ein therapeutisch inertes Excipiens.

**17.** Entzündungshemmendes und oedemhemmendes Mittel, enthaltend eine Verbindung gemäss einem der Ansprüche 1 bis 12 und ein therapeutisch inertes Excipiens.

**18.** Verwendung von Verbindungen gemäss einem der Ansprüche 1 bis 12 zur Herstellung von Arzneimitteln gegen Entzündungen und Oedeme.

**Patentansprüche für folgende Vertragsstaaten : ES, GR**

**1.** Verfahren zur Herstellung von Imidazolderivaten der allgemeinen Formel

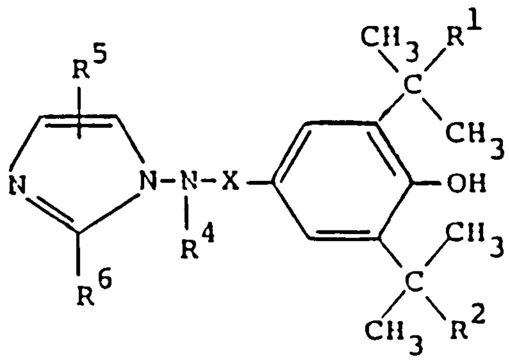

I

worin $R^1$ und $R^2$ je ($C_1$-$C_7$)-Alkyl, X einen Rest der Formel

$-CH(R^3)-$ oder $-CO-$ ;

(a) (b)

$R^3$ Wasserstoff und $R^4$ Wasserstoff oder ($C_1$-$C_7$)-Alkyl oder $R^3$ und $R^4$ zusammen eine zusätzliche Kohlenstoff-Stickstoff-Bindung; $R^5$ Wasserstoff oder ($C_1$-$C_7$)-Alkyl; $R^6$ Wasserstoff, ($C_1$-$C_7$)-Alkyl, ($C_1$-$C_7$)-Alkylthio oder einen Rest der Formel $-NR^7R^8$; und $R^7$ und $R^8$ je ($C_1$-$C_7$)-Alkyl oder zusammen mit dem Stickstoffatom einen 5- oder 6-gliedrigen gesättigten Heterocyclus bedeuten;
und von pharmazeutisch annehmbaren Säureadditionssalzen davon, dadurch gekennzeichnet, dass man

a) zur Herstellung einer Verbindung der Formel I, worin X $-CH(R^3)-$ und $R^3$ und $R^4$ zusammen eine zusätzliche Kohlenstoff-Stickstoff-Bindung bedeuten,
eine Verbindung der allgemeinen Formel

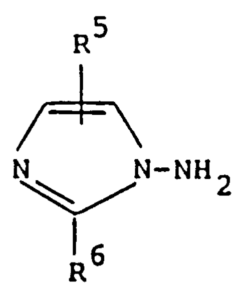

II

worin $R^5$ und $R^6$ obige Bedeutung besitzen,
mit einem Aldehyd der allgemeinen Formel

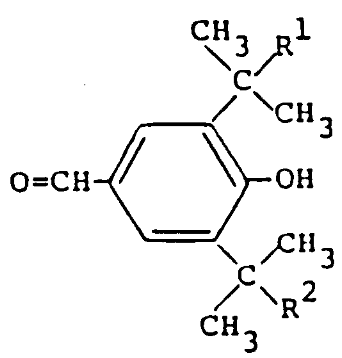

III

worin $R^1$ und $R^2$ obige Bedeutung besitzen,
kondensiert; oder
b) zur Herstellung einer Verbindung der Formel I, worin X -CO- und $R^4$ Wasserstoff oder ($C_1$-$C_7$)-Alkyl bedeuten,
eine Verbindung der allgemeinen Formel

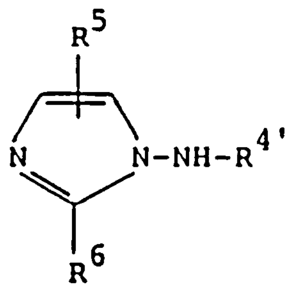

IV

worin $R^5$ und $R^6$ obige Bedeutung besitzen und $R^{4'}$ Wasserstoff oder ($C_1$-$C_7$)-Alkyl bedeutet,
mit einem reaktionsfähigen Derivat einer Carbonsäure der allgemeinen Formel

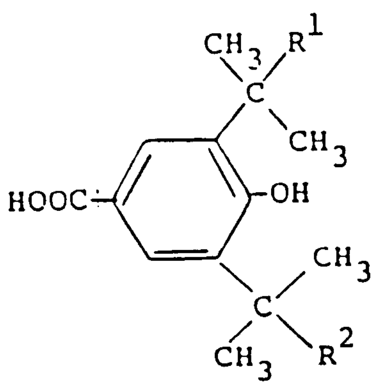

V

worin $R^1$ und $R^2$ obige Bedeutung besitzen,
acyliert; oder
c) zur Herstellung einer Verbindung der Formel I, worin X -$CH_2$- und $R^4$ Wasserstoff bedeuten,
eine Verbindung der allgemeinen Formel

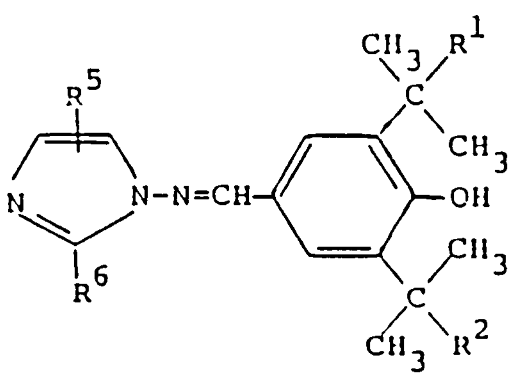

Ia

worin $R^1$, $R^2$, $R^5$ und $R^6$ obige Bedeutung besitzen,
reduziert; oder
d) zur Herstellung einer Verbindung der Formel I, worin X -$CH_2$- und $R^4$ Wasserstoff oder ($C_1$-$C_7$)-Alkyl bedeuten,
eine Verbindung der allgemeinen Formel

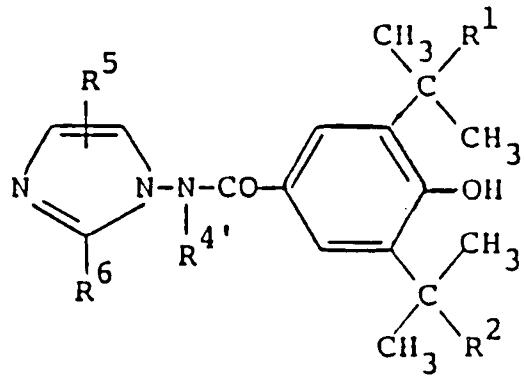

Ib

worin $R^1$, $R^2$, $R^5$, $R^6$ und $R^{4'}$ obige Bedeutung besitzen, reduziert; oder

e) zur Herstellung einer Verbindung der Formel I, worin X $-CH_2-$ und $R^4$ $(C_1-C_7)$-Alkyl bedeuten, eine Verbindung der allgemeinen Formel

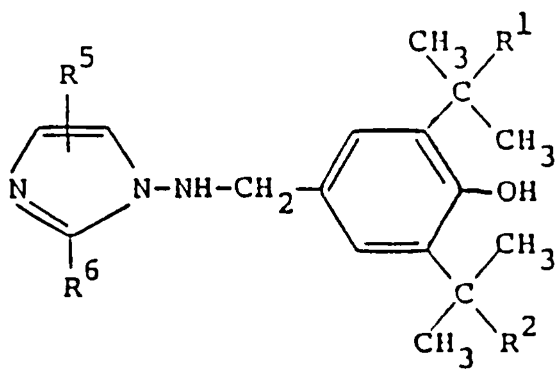

Ic

worin $R^1$, $R^2$, $R^5$ und $R^6$ obige Bedeutung besitzen, entsprechend N-alkyliert; oder

f) zur Herstellung einer Verbindung der Formel I, worin X $-CH(R^3)$ und $R^3$ und $R^4$ zusammen eine zusätzliche Kohlenstoff-Stickstoff-Bindung bedeuten, eine Verbindung der obigen Formel Ic dehydriert; oder

g) zur Herstellung einer Verbindung der Formel I, worin X -CO- oder $-CH_2-$, $R^4$ Wasserstoff oder $(C_1-C_7)$-Alkyl und $R^6$ Wasserstoff bedeuten, von einer Verbindung der allgemeinen Formel

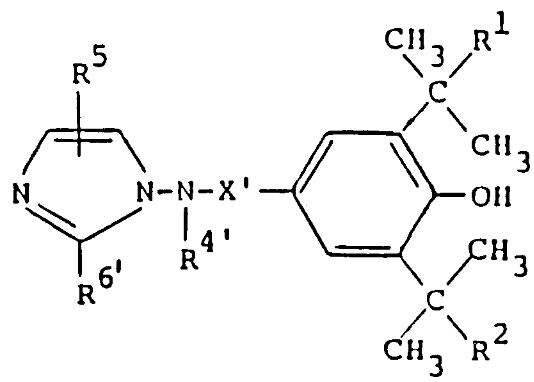

Id

worin $R^1$, $R^2$, $R^5$ und $R^{4'}$ obige Bedeutung besitzen und X' eine Methylen- oder Carbonylgruppe und $R^{6'}$ $(C_1-C_7)$-Alkylthio bedeuten,

die Alkylthiogruppe entfernt; oder
h) eine Verbindung der Formel I in ein pharmazeutisch annehmbares Säureadditionssalz überführt.

**2.** Verfahren nach Anspruch 1, dadurch gekennzeichnet, dass man eine Verbindung der Formel I herstellt, worin $R^1$ und $R^2$ je Methyl bedeuten.

**3.** Verfahren nach Anspruch 1 oder 2, dadurch gekennzeichnet, dass man eine Verbindung der Formel I herstellt, worin $R^5$ Wasserstoff oder Methyl in 4-Stellung bedeutet.

**4.** Verfahren nach einem der Ansprüche 1 bis 3, dadurch gekennzeichnet, dass man eine Verbindung der Formel I herstellt, worin $R^6$ Wasserstoff, Methyl, n-Propyl, Isopropyl, Methylmercapto oder Morpholino bedeutet.

**5.** Verfahren nach einem der Ansprüche 1 bis 4, dadurch gekennzeichnet, dass man eine Verbindung der Formel I herstellt, worin $R^3$ Wasserstoff und $R^4$ Wasserstoff oder Methyl oder $R^3$ und $R^4$ zusammen eine zusätzliche Kohlenstoff-Stickstoff-Bindung bedeuten.

**6.** Verfahren nach Anspruch 1, dadurch gekennzeichnet, dass man 1-(4-Hydroxy-3,5-di-tert.-butylbenzylamino)imidazol herstellt.

**7.** Verfahren nach Anspruch 1, dadurch gekennzeichnet, dass man 1-(4-Hydroxy-3,5-di-tert.-butylbenzylidenamino)-2-methylimidazol herstellt.

**8.** Verfahren nach Anspruch 1, dadurch gekennzeichnet, dass man 1-(4-Hydroxy-3,5-di-tert.-butylbenzylidenamino)imidazol herstellt.

**9.** Verfahren nach Anspruch 1, dadurch gekennzeichnet, dass man 1-(4-Hydroxy-3,5-di-tert.-butylbenzylamino)-2-methylimidazolherstellt.

**10.** Verfahren nach Anspruch 1, dadurch gekennzeichnet, dass man 1-(4-Hydroxy-3,5-di-tert.-butylbenzylamino)-2-propylimidazolherstellt.

**11.** Verfahren nach Anspruch 1, dadurch gekennzeichnet, dass man 1-(4-Hydroxy-3,5-di-tert.-butylbenzylamino)-2-methylmercaptoimidazol herstellt.

**12.** Verfahren zur Herstellung von Arzneimitteln, dadurch gekennzeichnet, dass man eine oder mehrere Verbindungen der in Anspruch 1 definierten Formel I oder pharmazeutisch annehmbare Säureadditionssalze davon und gegebenenfalls einen oder mehrere andere therapeutisch wertvolle Stoffe zusammen mit einem oder mehreren therapeutisch inerten Excipientien in eine galenische Darreichungsform bringt.

**13.** Verwendung von Verbindungen der in Anspruch 1 definierten Formel I und ihrer pharmazeutisch annehmbaren Säureadditionssalze zur Herstellung von Arzneimitteln gegen Entzündungen und Oedeme.

**Claims**
**Claims for the following Contracting States : AT, BE, CH, DE, FR, GB, IT, LI, LU, NL, SE**

**1.** Imidazole derivatives of the general formula

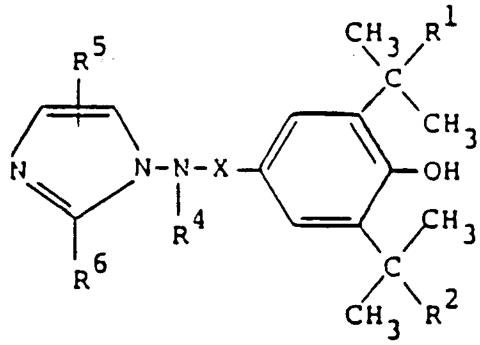

I

wherein $R^1$ and $R^2$ each signify $(C_1-C_7)$-alkyl, X signifies a residue of the formula

$$-CH(R^3)- \quad \text{or} \quad -CO- \;;$$

(a) (b)

$R^3$ signifies hydrogen and $R^4$ signifies hydrogen or $(C_1-C_7)$-alkyl or $R^3$ and $R^4$ together signify an additional carbon-nitrogen bond; $R^5$ signifies hydrogen or $(C_1-C_7)$-alkyl; $R^6$ signifies hydrogen, $(C_1-C_7)$-alkyl, $(C_1-C_7)$-alkylthio or a residue of the formula $-NR^7R^8$; and $R^7$ and $R^8$ each signify $(C_1-C_7)$-alkyl or together with the nitrogen atom signify a 5- or 6-membered saturated heterocycle;
and pharmaceutically acceptable acid addition salts thereof.

**2.** Compounds according to claim 1, wherein $R^1$ and $R^2$ each signify methyl.

**3.** Compounds according to claim 1 or 2, wherein $R^5$ signifies hydrogen or methyl in the 4-position.

**4.** Compounds according to any one of claims 1 to 3, wherein $R^6$ signifies hydrogen, methyl, n-propyl, isopropyl, methylmercapto or morpholino.

**5.** Compounds according to any one of claims 1 to 5, wherein $R^3$ signifies hydrogen and $R^4$ signifies hydrogen or methyl or $R^3$ and $R^4$ together signify an additional carbon-nitrogen bond.

**6.** 1-(4-Hydroxy-3,5-di-tert.-butylbenzylamino)-imidazole.

**7.** 1-(4-Hydroxy-3,5-di-tert.-butylbenzylideneamino)-2-methylimidazole.

**8.** 1-(4-Hydroxy-3,5-di-tert.-butylbenzylideneamino)-imidazole.

**9.** 1-(4-Hydroxy-3,5-di-tert.-butylbenzylamino)-2-methylimidazole.

**10.** 1-(4-Hydroxy-3,5-di-tert.-butylbenzylamino)-2-propylimidazole.

**11.** 1-(4-Hydroxy-3,5-di-tert.-butylbenzylamino)-2-methylmercaptoimidazole.

**12.** 1-(4-Hydroxy-3,5-di-tert.-butylbenzoylamino)-2-methylimidazole;
1-(4-hydroxy-3,5-di-tert.-butylbenzoylamino)imidazole;
1-(4-hydroxy-3,5-di-tert.-butylbenzylideneamino)-2-morpholinoimidazole;
1-(4-hydroxy-3,5-di-tert.-butylbenzylamino)-2-morpholinoimidazole;
1-(4-hydroxy-3,5-di-tert.-butylbenzylideneamino)-2-isopropylimidazole;
1-(4-hydroxy-3,5-di-tert.-butylbenzylideneamino)-4-methylimidazole;
1-(4-hydroxy-3,5-di-tert.-butylbenzylamino)-4-methylimidazole;
1-(4-hydroxy-3,5-di-tert.-butylbenzylideneamino)-2-propylimidazole; and

1-[N-(4-hydroxy-3,5-di-tert.-butylbenzyl)-N-methyl-amino]imidazole.

**13.** Compounds in accordance with any one of claims 1 to 12 for use as therapeutically active substances.

**14.** Compounds in accordance with any one of claims 1 to 12 for use as inflammation-inhibiting and oedema-inhibiting active substances.

**15.** A process for the manufacture of compounds of formula I defined in claim I and of pharmaceutically acceptable acid addition salts thereof, characterized by

a) for the manufacture of a compound of formula I in which X signifies -CH($R^3$)- and $R^3$ and $R^4$ together signify an additional carbon-nitrogen bond, condensing a compound of the general formula

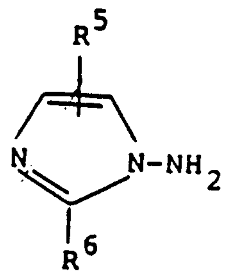

II

wherein $R^5$ and $R^6$ have the significance given in claim 1,
with an aldehyde of the general formula

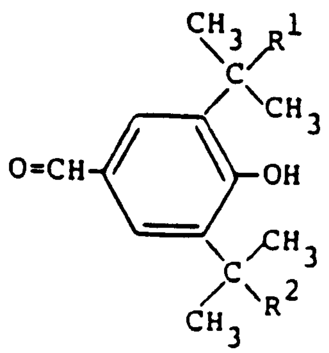

III

wherein $R^1$ and $R^2$ have the significance given in claim 1;

or

b) for the manufacture of a compound of formula I in which X signifies -CO- and $R^4$ signifies hydrogen or ($C_1$-$C_7$)-alkyl, acylating a compound of the general formula

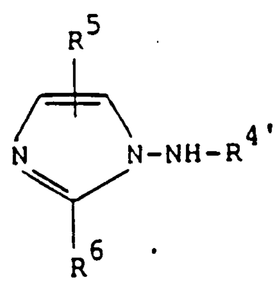

IV

wherein $R^5$ and $R^6$ have the significance given in claim 1 and $R^{4'}$ signifies hydrogen or ($C_1$-$C_7$)-alkyl, with a reactive derivative of a carboxylic acid of the general formula

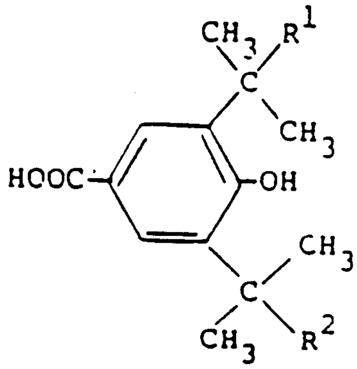

V

wherein $R^1$ and $R^2$ have the significance given in claim 1;
or
c) for the manufacture of a compound of formula I in which X signifies $-CH_2-$ and $R^4$ signifies hydrogen,
reducing a compound of the general formula

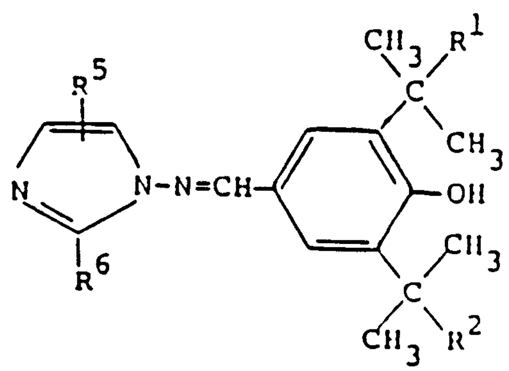

Ia

wherein $R^1$, $R^2$, $R^5$ and $R^6$ have the significance given in claim 1;
or
d) for the manufacture of a compound of formula I in which X signifies $-CH_2-$ and $R^4$ signifies hydrogen or $(C_1\text{-}C_7)$-alkyl, reducing a compound of the general formula

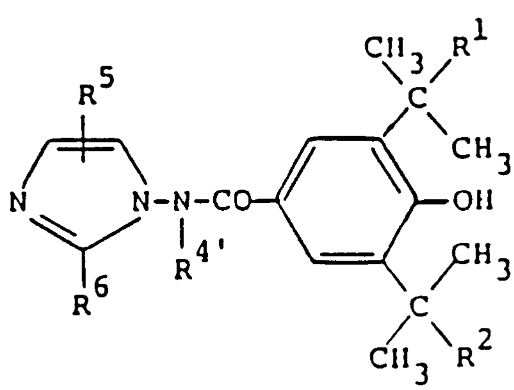

Ib

wherein $R^1$, $R^2$, $R^5$ and $R^6$ have the significance given in claim 1 and $R^{4'}$ has the above significance;
or
e) for the manufacture of a compound of formula I in which X signifies $-CH_2-$ and $R^4$ signifies $(C_1\text{-}C_7)$-alkyl, appropriately N-alkylating a compound of the general formula

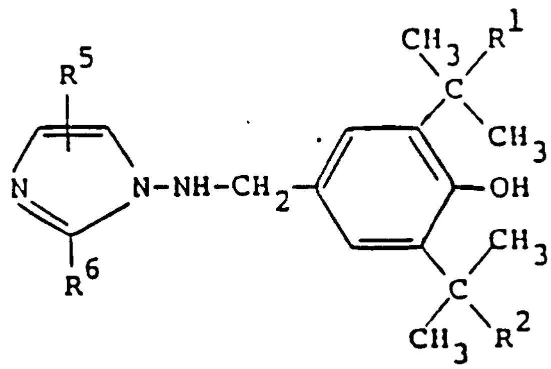

Ic

wherein $R^1$, $R^2$, $R^5$ and $R^6$ have the significance given in claim 1;
or
f) for the manufacture of a compound of formula I in which X signifies -CH($R^3$)- and $R^3$ and $R^4$ together signify an additional carbon-nitrogen bond, dehydrogenating a compound of formula Ic above; or
g) for the manufacture of a compound of formula I in which X signifies -CO- or -$CH_2$-, $R^4$ signifies hydrogen or ($C_1$-$C_7$)-alkyl and $R^6$ signifies hydrogen, removing the alkylthio group from a compound of the general formula

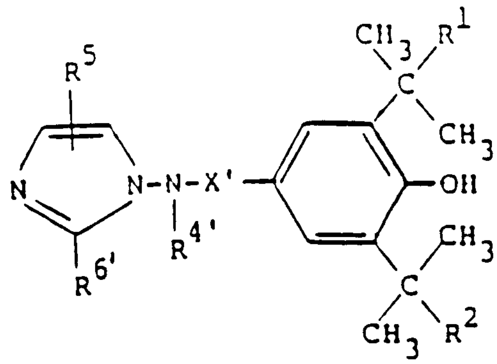

Id

wherein $R^1$, $R^2$, and $R^5$ have the significance given in claim 1 and $R^{4'}$ has the above significance and X' signifies a methylene or carbonyl group and $R^{6'}$ signifies ($C_1$-$C_7$)- alkylthio;
or
h) converting a compound of formula I into a pharmaceutically acceptable acid addition salt.

**16.** A medicament containing a compound in accordance with any one of claims 1 to 12 and a therapeutically inert excipient.

**17.** An inflammation-inhibiting and oedema-inhibiting medicament, containing a compound in accordance with any one of claims 1 to 12 and a therapeutically inert excipient.

**18.** The use of compounds in accordance with any one of claims 1 to 12 for the manufacture of medicaments against inflammations and oedemas.

**Claims for the following Contracting States : ES, GR**

**1.** A process for the manufacture of imidazole derivatives of the general formula

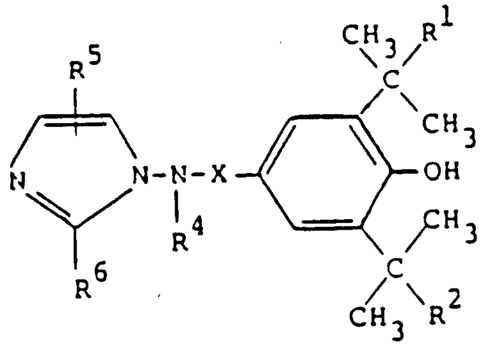

I

wherein $R^1$ and $R^2$ each signify ($C_1$-$C_7$)-alkyl, X signifies a residue of the formula

$-CH(R^3)-$ (a) or $-CO-$ (b) ;

$R^3$ signifies hydrogen and $R^4$ signifies hydrogen or ($C_1$-$C_7$)-alkyl or $R^3$ and $R^4$ together signify an additional carbon-nitrogen bond; $R^5$ signifies hydrogen or ($C_1$-$C_7$)-alkyl; $R^6$ signifies hydrogen, ($C_1$-$C_7$)-alkyl, ($C_1$-$C_7$)-alkythio or a residue of the formula $-NR^7R^8$; and $R^7$ and $R^8$ each signify ($C_1$-$C_7$)-alkyl or together with the nitrogen atom signify a 5- or 6-membered saturated heterocycle;
and pharmaceutically acceptable acid addition salts thereof, characterized by

a) for the manufacture of a compound of formula I in which X signifies $-CH(R^3)-$ and $R^3$ and $R^4$ together signify an additional carbon-nitrogen bond, condensing a compound of the general formula

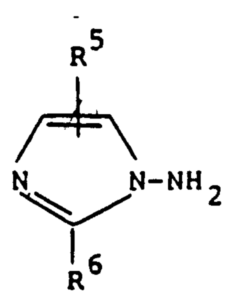

II

wherein $R^5$ and $R^6$ have the significance given above,
with an aldehyde of the general formula

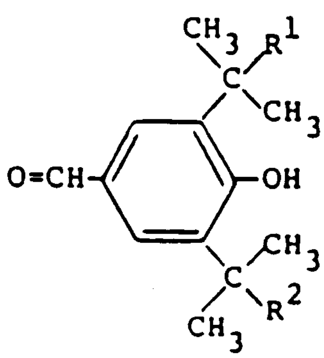

III

wherein $R^1$ and $R^2$ have the significance given above;
or
b) for the manufacture of a compound of formula I in which X signifies -CO- and $R^4$ signifies hydrogen or ($C_1$-$C_7$)-alkyl, acylating a compound of the general formula

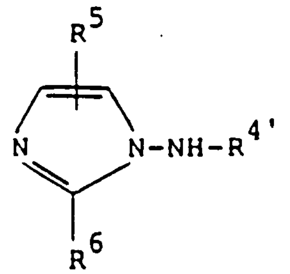

IV

wherein $R^5$ and $R^6$ have the significance given above and $R^{4'}$ signifies hydrogen or ($C_1$-$C_7$)- alkyl, with a reactive derivative of a carboxylic acid of the general formula

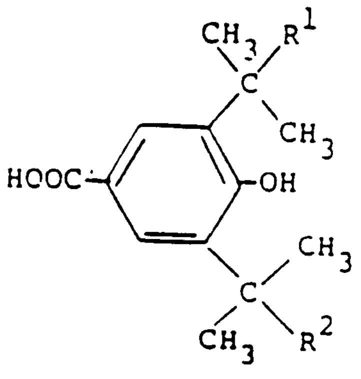

V

wherein $R^1$ and $R^2$ have the significance given above;
or
c) for the manufacture of a compound of formula I in which X signifies $-CH_2-$ and $R^4$ signifies hydrogen, reducing a compound of the general formula

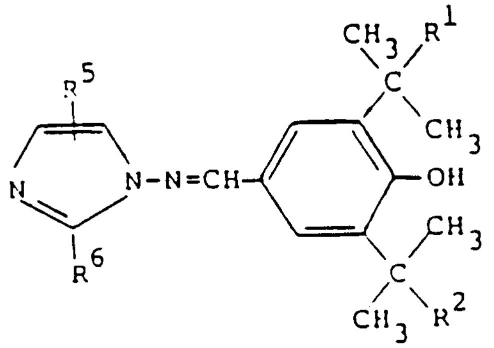

Ia

wherein $R^1$, $R^2$, $R^5$ and $R^6$ have the significance given above;
or
d) for the manufacture of a compound of formula I in which X signifies $-CH_2-$ and $R^4$ signifies hydrogen or ($C_1$-$C_7$)-alkyl, reducing a compound of the general formula

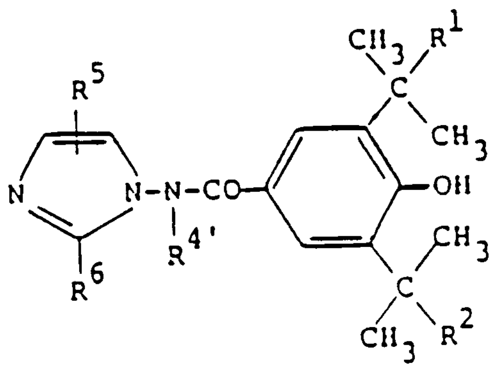

Ib

wherein $R^1$, $R^2$, $R^5$, $R^6$ and $R^{4'}$ have the significance given above;
or

e) for the manufacture of a compound of formula I in which X signifies $-CH_2-$ and $R^4$ signifies $(C_1-C_7)$-alkyl, appropriately N-alkylating a compound of the general formula

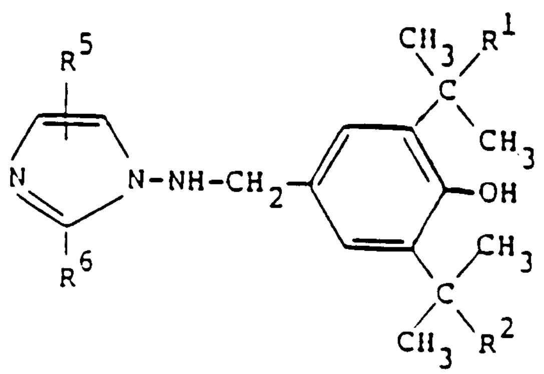

Ic

wherein $R^1$, $R^2$, $R^5$ and $R^6$ have the significance given above;
or

f) for the manufacture of a compound of formula I in which X signifies $-CH(R^3)-$ and $R^3$ and $R^4$ together signify an additional carbon-nitrogen bond, dehydrogenating a compound of formula Ic above; or

g) for the manufacture of a compound of formula I in which X signifies -CO- or $-CH_2-$, $R^4$ signifies hydrogen or $(C_1-C_7)$-alkyl and $R^6$ signifies hydrogen, removing the alkylthio group from a compound of the general formula

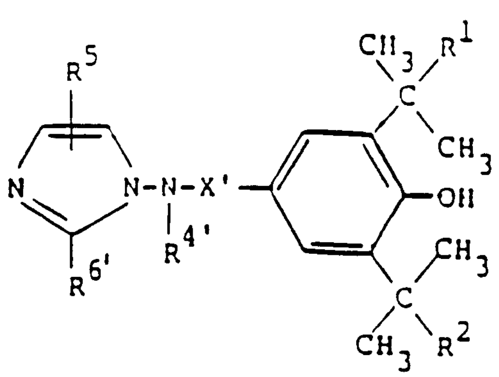

Id

wherein $R^1$, $R^2$, $R^5$ and $R^{4'}$ have the sicance given above, X' signifies a methylene or carbonyl group and $R^{6'}$ signifies $(C_1-C_7)$-alkylthio;
or

h) converting a compound of formula I into a pharmaceutically acceptable acid addition salt.

2. A process according to claim 1, characterized in that a compound of formula I in which $R^1$ and $R^2$ each signify methyl is manufactured.

3. A process according to claim 1 or 2, characterized in that a compound of formula I in which $R^5$ signifies hydrogen or methyl in the 4-position is manufactured.

4. A process according to any one of claims 1 to 3, characterized in that a compound of formula I in which $R^6$ signifies hydrogen, methyl, n-propyl, isopropyl, methylmercapto or moropholino is manufactured.

5. A process according to any one of claims 1 to 4, characterized in that a compound of formula I in which $R^3$ signifies hydrogen and $R^4$ signifies hydrogen or methyl or $R^3$ and $R^4$ together signify an additional carbon-nitrogen bond is manufactured.

6. A process according to claim 1, characterized in that 1-(4-hydroxy-3,5-di-tert.-butylbenzylamino)-imidazole is manufactured.

7. A process according to claim 1, characterized in that 1-(4-hydroxy-3,5-di-tert.-butylbenzylideneamino)-2-methylimidazole is manufactured.

8. A process according to claim 1, characterized in that 1-(4-hydroxy-3,5-di-tert.-butylbenzylideneamino)-imidazole is manufactured.

9. A process according to claim 1, characterized in that 1-(4-hydroxy-3,5-di-tert.-butylbenzylamino)-2-methylimidazole is manufactured.

10. A process according to claim 1, characterized in that 1-(4-hydroxy-3,5-di-tert.-butylbenzylamino)-2-propylimidazole is manufactured.

11. A process according to claim 1, characterized in that 1-(4-hydroxy-3,5-di-tert.-butylbenzylamino)-2-methylmercaptoimidazole is manufactured.

12. A process for the manufacture of medicaments, characterized by bringing one or more compounds of formula I as defined in claim 1 or pharmaceutically acceptable acid addition salts thereof and, if desired, one or more other therapeutically valuable substances into a galenical administration form together with one or more therapeutically inert excipients.

13. The use of compounds of formula I as defined in claim 1 and of pharmaceutically acceptable acid addition salts thereof for the manufacture of medicaments against inflammations and oedemas.

**Revendications**

**Revendications pour les Etats contractants suivants : AT, BE, CH, DE, FR, GB, IT, LI, LU, NL, SE**

1. Dérivés de l'imidazole de formule générale

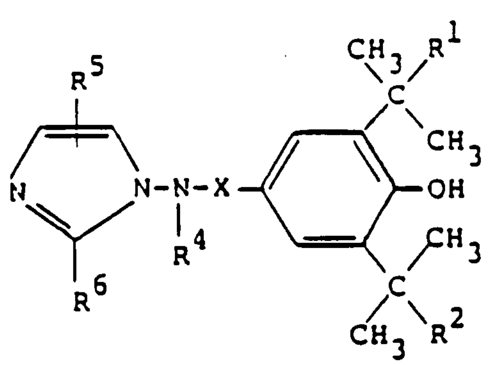

I

dans laquelle $R^1$ et $R^2$ représentent chacun un groupe alkyle en $C_1$ -$C_7$ , X est un groupe de formule

$$\underset{(a)}{-CH(R^3)-} \quad \text{ou} \quad \underset{(b)}{-CO-} \quad ;$$

$R^3$ représente l'hydrogène et $R^4$ l'hydrogène ou un groupe alkyle en $C_1$ -$C_7$ , ou bien $R^3$ et $R^4$ forment ensemble une liaison carbone-azote supplémentaire; $R^5$ représente l'hydrogène ou un groupe alkyle en $C_1$ -$C_7$ ; $R^6$ représente l'hydrogène, un groupe alkyle en $C_1$ -$C_7$ , alkylthio en $C_1$ -$C_7$ ou un groupe de formule $-NR^7R^8$ dans lequel $R^7$ et $R^8$ représentent chacun un groupe alkyle en $C_1$ -$C_7$ ou forment ensemble et avec l'atome d'azote un hétérocycle saturé à 5 ou 6 chaînons;
et leurs sels formés par addition avec des acides acceptables pour l'usage pharmaceutique.

**2.** Composés selon la revendication 1, dans lesquels $R^1$ et $R^2$ représentent chacun un groupe méthyle.

**3.** Composés selon la revendication 1 ou 2, dans lesquels $R^5$ représente l'hydrogène ou un groupe méthyle en position 4.

**4.** Composés selon l'une des revendications 1 à 3, dans lesquels $R^6$ représente l'hydrogène, un groupe méthyle, n-propyle, isopropyle, méthylmercapto ou morpholino.

**5.** Composés selon l'une des revendications 1 à 4, dans lesquels $R^3$ représente l'hydrogène et $R^4$ l'hydrogène ou un groupe méthyle, ou bien $R^3$ et $R^4$ forment ensemble une liaison carbone-azote supplémentaire.

**6.** Le 1-(4-hydroxy-3,5-di-tert-butylbenzylamino)-imidazole.

**7.** Le 1-(4-hydroxy-3,5-di-tert-butylbenzylidèneamino)-2-méthylimidazole.

**8.** Le 1-(4-hydroxy-3,5-di-tert-butylbenzylidèneamino)-imidazole.

**9.** Le 1-(4-hydroxy-3,5-di-tert-butylbenzylamino)-2-méthylimidazole.

**10.** Le 1-(4-hydroxy-3,5-di-tert-butylbenzylamino)-2-propylimidazole.

**11.** Le 1-(4-hydroxy-3,5-di-tert-butylbenzylamino)-2-méthylmercaptoimidazole.

**12.** Le 1-(4-hydroxy-3,5-di-tert-butylbenzoylamino)-2-méthylimidazole;
le 1-(4-hydroxy-3,5-di-tert-butylbenzoylamino)imidazole;
le 1-(4-hydroxy-3,5-di-tert-butylbenzylidène-amino)-2-morpholinoimidazole;
le 1-(4-hydroxy-3,5-di-tert-butylbenzylamino)-2-morpholinoimidazole;
le 1-(4-hydroxy-3,5-di-tert-butylbenzylidène-amino)-2-isopropylimidazole;
le 1-(4-hydroxy-3,5-di-tert-butylbenzylidène-amino)-4-méthylimidazole;
le 1-(4-hydroxy-3,5-di-tert-butylbenzylamino)-4-méthylimidazole;
le 1-(4-hydroxy-3,5-di-tert-butylbenzylidène-amino)-2-propylimidazole; et
le 1-[N-(4-hydroxy-3,5-di-tert-butylbenzyl)-N-méthylamino]imidazole

**13.** Composés selon l'une des revendications 1 à 12, pour l'utilisation en tant que substances actives thérapeutiques.

**14.** Composés selon l'une des revendications 1 à 12, pour l'utilisation en tant que substances actives anti-inflammatoires et anti-oedèmes.

**15.** Procédé de préparation des composés de formule I de la revendication 1 et de leurs sels formés par addition avec des acides acceptables pour l'usage pharmaceutique, caractérisé en ce que :
a) pour la préparation d'un composé de formule I dans laquelle X représente -CH($R^3$)- et $R^3$ et $R^4$ forment ensemble une liaison carbone-azote supplémentaire,

on condense un composé de formule générale

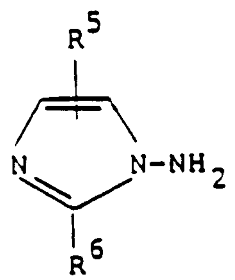

II

dans laquelle $R^5$ et $R^6$ ont les significations indiquées dans la revendication 1, avec un aldéhyde de formule générale

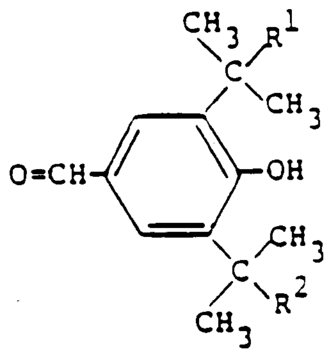

III

dans laquelle $R^1$ et $R^2$ ont les significations indiquées dans la revendication 1, ou bien

b) pour préparer un composé de formule I dans laquelle X représente -CO- et $R^4$ représente l'hydrogène ou un groupe alkyle en $C_1$ -$C_7$ ,

on acyle un composé de formule générale

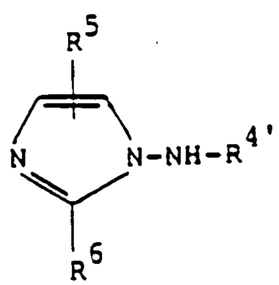

IV

dans laquelle $R^5$ et $R^6$ ont les significations indiquées dans la revendication 1, et $R^{4'}$ représente l'hydrogène ou un groupe alkyle en $C_1$ -$C_7$ ,

à l'aide d'un dérivé réactif d'un acide carboxylique de formule générale

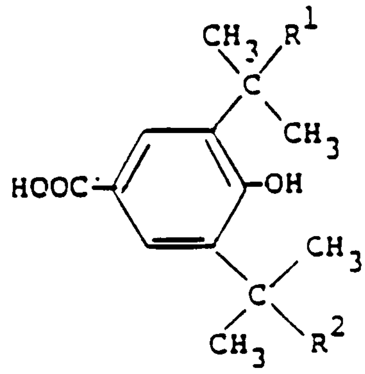

V

dans laquelle $R^1$ et $R^2$ ont les significations indiquées dans la revendication 1, ou bien
c) pour préparer un composé de formule I dans laquelle X représente $-CH_2-$ et $R^4$ l'hydrogène, on réduit un composé de formule générale

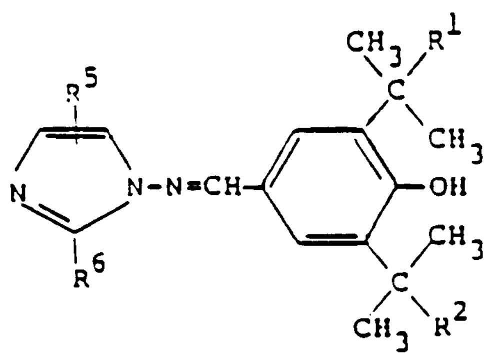

Ia

dans laquelle $R^1$, $R^2$, $R^5$ et $R^6$ ont les significations indiquées dans la revendication 1, ou bien
d) pour préparer un composé de formule I dans laquelle X représente $-CH_2-$ et $R^4$ l'hydrogène ou un groupe alkyle en $C_1$ -$C_7$ ,
on réduit un composé de formule générale

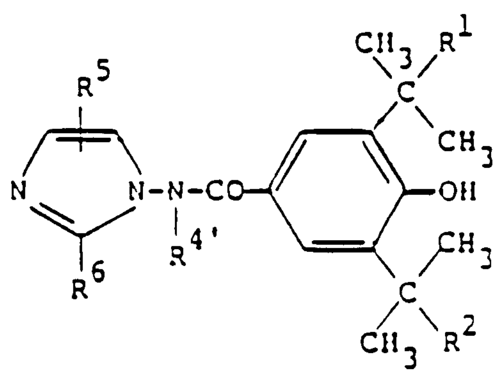

Ib

dans laquelle $R^1$, $R^2$, $R^5$ et $R^6$ ont les significations indiquées dans la revendication 1 et $R^{4'}$ la signification indiquée ci-dessus, ou bien
e) pour préparer un composé de formule I dans laquelle X représente $-CH_2-$ et $R^4$ un groupe alkyle en $C_1$ -$C_7$ ,
on soumet un composé de formule générale

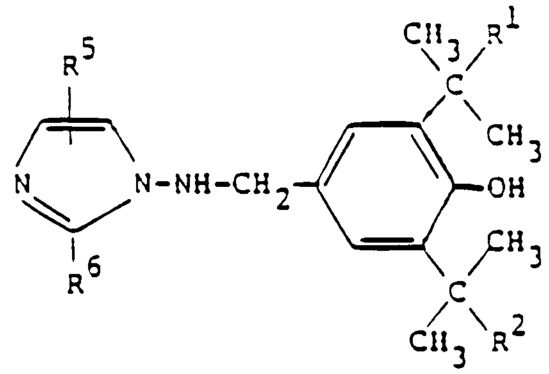

Ic

dans laquelle $R^1$, $R^2$, $R^5$ et $R^6$ ont les significations indiquées dans la revendication 1, à une N-alkylation appropriée; ou bien

f) pour préparer un composé de formule I dans laquelle X représente $-CH(R^3)-$ et $R^3$ et $R^4$ forment ensemble une liaison carbone-azote supplémentaire, on soumet un composé de formule Ic ci-dessus à déshydrogénation; ou bien

g) pour préparer un composé de formule I dans laquelle X représente -CO- ou $-CH_2-$, $R^4$ l'hydrogène ou un groupe alkyle en $C_1$ -$C_7$ et $R^6$ l'hydrogène, partant d'un composé de formule générale

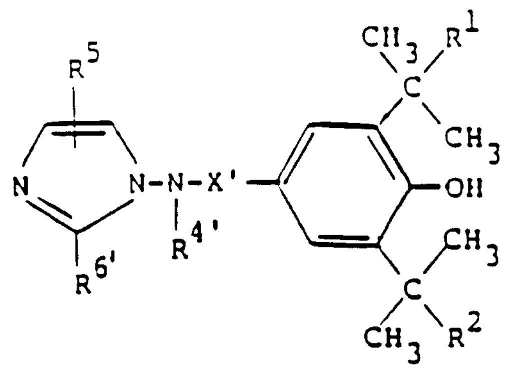

Id

dans laquelle $R^1$, $R^2$ et $R^5$ ont les significations indiquées dans la revendication 1, $R^{4'}$ la signification indiquée ci-dessus, X' représente un groupe méthylène ou carbonyle et $R^{6'}$ représente un groupe alkylthio en $C_1$ -$C_7$,

on élimine le groupe alkylthio; ou bien

h) on convertit un composé de formule I en un sel formé par addition avec un acide acceptable pour l'usage pharmaceutique.

**16.** Médicament contenant un composé selon l'une des revendications 1 à 12 et un excipient inerte du point de vue thérapeutique.

**17.** Agent anti-inflammatoire et anti-oedème contenant un composé selon l'une des revendications 1 à 12 et un excipient inerte du point de vue thérapeutique.

**18.** Utilisation de composés selon l'une des revendications 1 à 12 pour la préparation de médicaments contre les inflammations et les oedèmes.

**Revendications pour les Etats contractants suivants : ES, GR**

**1.** Procédé de préparation de dérivés de l'imidazole répondant à la formule générale

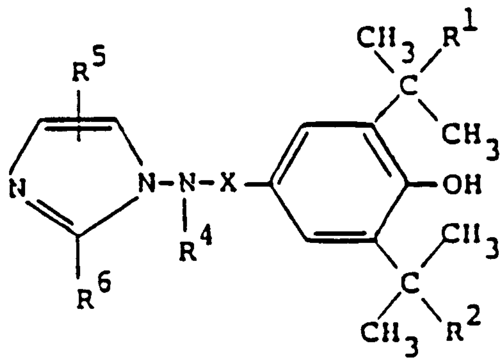

I

dans laquelle $R^1$ et $R^2$ représentent chacun un groupe alkyle en $C_1$ -$C_7$ , X représente un groupe de formule

| | | | |
|---|---|---|---|
| $-CH(R^3)-$ | ou | $-CO-$ | ; |
| (a) | | (b) | |

$R^3$ représente l'hydrogène et $R^4$ l'hydrogène ou un groupe alkyle en $C_1$ -$C_7$ , ou bien $R^3$ et $R^4$ forment ensemble une liaison supplémentaire carbone-azote; $R^5$ représente l'hydrogène ou un groupe alkyle en $C_1$ -$C_7$ ; $R^6$ représente l'hydrogène, un groupe alkyle en $C_1$ -$C_7$ , alkylthio en $C_1$ -$C_7$ ou un groupe de formule $-NR^7R^8$ dans lequel $R^7$ et $R^8$ représentent chacun un groupe alkyle en $C_1$ -$C_7$ ou forment ensemble et avec l'atome d'azote un hétérocycle saturé à 5 ou 6 chaînons,
et de leurs sels formés par addition avec des acides acceptables pour l'usage pharmaceutique, caractérisé en ce que :

a) pour la préparation d'un composé de formule I dans laquelle X représente $-CH(R^3)-$ et $R^3$ et $R^4$ forment ensemble une liaison carbone-azote supplémentaire,
on condense un composé de formule générale

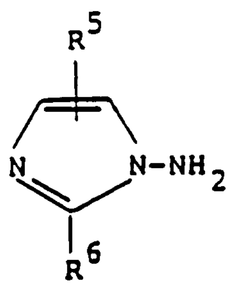

II

dans laquelle $R^5$ et $R^6$ ont les significations indiquées ci-dessus,
avec un aldéhyde de formule générale

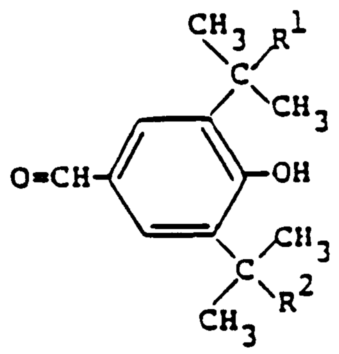

III

dans laquelle $R^1$ et $R^2$ ont les significations indiquées ci-dessus; ou bien

b) pour préparer un composé de formule I dans laquelle X représente -CO- et $R^4$ l'hydrogène ou un groupe alkyle en $C_1$ -$C_7$ ,

on acyle un composé de formule générale

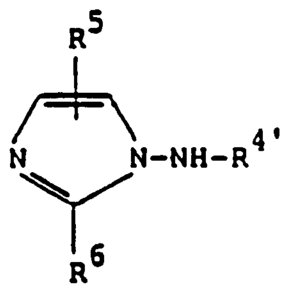

IV

dans laquelle $R^5$ et $R^6$ ont les significations indiquées ci-dessus, et $R^{4'}$ représente l'hydrogène ou un groupe alkyle en $C_1$ -$C_7$ ,

à l'aide d'un dérivé réactif d'un acide carboxylique de formule générale

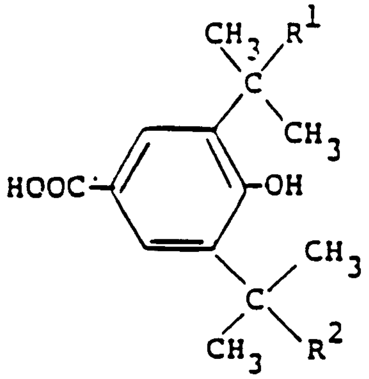

V

dans laquelle $R^1$ et $R^2$ ont les significations indiquées ci-dessus; ou bien

c) pour préparer un composé de formule I dans laquelle X représente -$CH_2$- et $R^4$ l'hydrogène,

on réduit un composé de formule générale

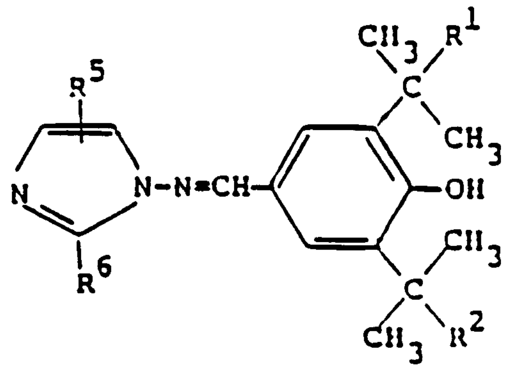

Ia

dans laquelle $R^1$, $R^2$, $R^5$ et $R^6$ ont les significations indiquées ci-dessus; ou bien

d) pour préparer un composé de formule I dans laquelle X représente $-CH_2-$ et $R^4$ l'hydrogène ou un groupe alkyle en $C_1$ -$C_7$ ,

on réduit un composé de formule générale

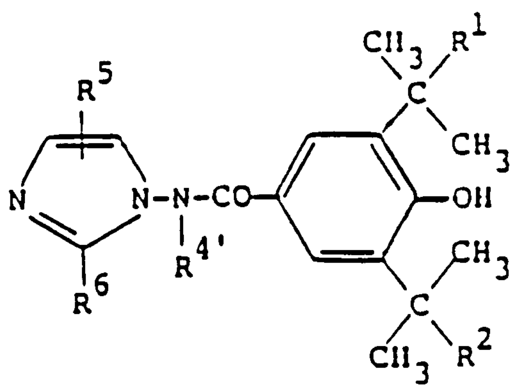

Ib

dans laquelle $R^1$, $R^2$, $R^5$ et $R^6$ et $R^{4'}$ ont les significations indiquées ci-dessus, ou bien

e) pour préparer un composé de formule I dans laquelle X représente $-CH_2-$ et $R^4$ un groupe alkyle en $C_1$ -$C_7$ ,

on soumet un composé de formule générale

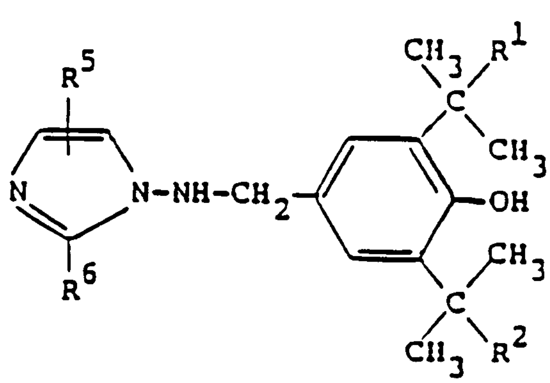

Ic

dans laquelle $R^1$, $R^2$, $R^5$ et $R^6$ ont les significations indiquées ci-dessus,

à une N-alkylation appropriée; ou bien

f) pour préparer un composé de formule I dans laquelle X représente $-CH(R^3)-$ et $R^3$ et $R^4$ forment ensemble une liaison carbone-azote supplémentaire,

on soumet un composé de formule Ic ci-dessus à déshydrogénation; ou bien

g) pour préparer un composé de formule I dans laquelle X représente -CO- ou $-CH_2-$, $R^4$ l'hydrogène ou un groupe alkyle en $C_1$ -$C_7$ et $R^6$ l'hydrogène,

partant d'un composé de formule générale

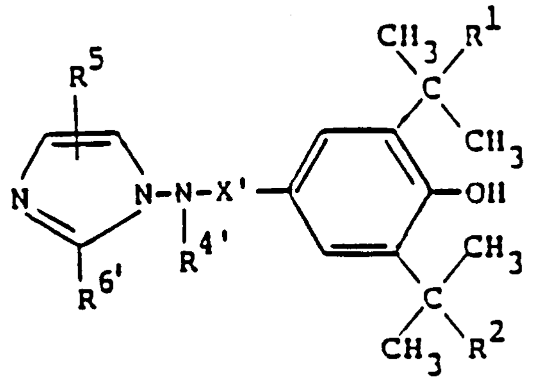

Id

dans laquelle $R^1$, $R^2$, $R^5$ et $R^{4'}$ ont les significations indiquées ci-dessus, X' représente un groupe méthylène ou carbonyle et $R^{6'}$ un groupe alkylthio en $C_1$ -$C_7$ ,
on élimine le groupe alkylthio; ou bien
h) on convertit un composé de formule I en un sel formé par addition avec un acide acceptable pour l'usage pharmaceutique.

**2.** Procédé selon la revendication 1, caractérisé en ce que l'on prépare un composé de formule I dans laquelle $R^1$ et $R^2$ représentent chacun un groupe méthyle.

**3.** Procédé selon la revendication 1 ou 2, caractérisé en ce que l'on prépare un composé de formule I dans laquelle $R^5$ représente l'hydrogène ou un groupe méthyle en position 4.

**4.** Procédé selon l'une des revendications 1 à 3, caractérisé en ce que l'on prépare un composé de formule I dans laquelle $R^6$ représente l'hydrogène, un groupe méthyle, n-propyle, isopropyle, méthylmercapto ou morpholino.

**5.** Procédé selon l'une des revendications 1 à 4, caractérisé en ce que l'on prépare un composé de formule I dans laquelle $R^3$ représente l'hydrogène et $R^4$ l'hydrogène ou un groupe méthyle ou bien $R^3$ et $R^4$ forment ensemble une liaison carbone-azote supplémentaire.

**6.** Procédé selon la revendication 1, caractérisé en ce que l'on prépare le 1-(4-hydroxy-3,5-di-tert-butylbenzylamino)imidazole.

**7.** Procédé selon la revendication 1, caractérisé en ce que l'on prépare le 1-(4-hydroxy-3,5-di-tert-butylbenzylidène-amino)-2-méthylimidazole.

**8.** Procédé selon la revendication 1, caractérisé en ce que l'on prépare le 1-(4-hydroxy-3,5-di-tert-butylbenzylidène-amino)imidazole.

**9.** Procédé selon la revendication 1, caractérisé en ce que l'on prépare le 1-(4-hydroxy-3,5-di-tert-butylbenzylamino)-2-méthylimidazole.

**10.** Procédé selon la revendication 1, caractérisé en ce que l'on prépare le 1-(4-hydroxy-3,5-di-tert-butylbenzylamino)-2-propylimidazole.

**11.** Procédé selon la revendication 1, caractérisé en ce que l'on prépare le 1-(4-hydroxy-3,5-di-tert-butylbenzylamino)-2-méthylmercaptoimidazole.

**12.** Procédé de préparation de médicaments, caractérisé en ce que l'on met un ou plusieurs composés de formule I de la revendication 1 ou leurs sels formés par addition avec des acides acceptables pour l'usage pharmaceutique et le cas échéant une ou plusieurs autres substances ayant une activité thérapeutique, avec un ou plusieurs excipients inertes du point de vue thérapeutique, sous une forme

d'administration galénique.

**13.** Utilisation des composés de formule I de la revendication 1 et de leurs sels formés par addition avec des acides acceptables pour l'usage pharmaceutique pour la préparation de médicaments contre les inflammations et les oedèmes.